# EUROPÄISCHE PATENTSCHRIFT

(11) **EP 1 409 745 B1**
(45) Veröffentlichungstag und Bekanntmachung des Hinweises auf die Patenterteilung: **11.04.2007**
(21) Anmeldenummer: 02772273.5
(22) Anmeldetag: 06.09.2002
(51) Int. Cl.: C12Q 1/68

(54) **VERFAHREN UND KIT ZUR DIAGNOSTIK ODER BEHANDLUNGSKONTROLLE VON DARMKREBS**
METHOD AND KIT FOR THE DIAGNOSIS OR TREATMENT CONTROL OF INTESTINAL CARCINOMA
PROCEDE ET NECESSAIRE DESTINES AU DIAGNOSTIC OU AU CONTROLE DU TRAITEMENT DU CANCER DE L'INTESTIN

(30) Priorität: 06.09.2001 DE 10143775; 17.05.2002 WO PCT/EP02/05489
(43) Veröffentlichungstag der Anmeldung: 21.04.2004
(73) Patentinhaber: Adnagen AG, 30853 Hannover-Langenhagen (DE)
(72) Erfinder: ALBERT, Winfried, 82377 Penzberg (DE); STEFFENS, Pia, D- 30177 Hannover (DE); KREHAN, Alf-Andreas, 82515 Heidelberg (DE); HECHT, Monica, 31515 Wunstorf-Kolenfeld (DE); WASCHÜTZA, Stefanie, 30169 Hannover (DE)
(74) Vertreter: Pfenning, Meinig & Partner GbR
(86) Internationale Anmeldenummer: PCT/EP2002/009983
(87) Internationale Veröffentlichungsnummer: WO 2003/023059

(56) Entgegenhaltungen:
- WO-A-94/07139
- WO-A-97/37226
- WO-A-99/44064
- DE-A- 19 736 691
- SMITH B ET AL: "DETECTION OF MELANOMA CELLS IN PERIPHERAL BLOOD BY MEANS OF REVERSE TRANSCRIPTASE AND POLYMERASE CHAIN REACTION" LANCET THE, LANCET LIMITED. LONDON, GB, Bd. 338, 16. November 1991 (1991-11-16), Seiten 1227-1229, XP002008751 ISSN: 0140-6736
- GHOSSEIN R A ET AL: "Molecular detection of micrometastases and circulating tumor cells in solid tumors" CLINICAL CANCER RESEARCH, THE AMERICAN ASSOCIATION FOR CANCER RESEARCH, US, Bd. 5, Nr. 8, August 1999 (1999-08), Seiten 1950-1960, XP002225510 ISSN: 1078-0432
- HARDINGHAM J E ET AL: "IMMUNOBEAD-PCR: A TECHNIQUE FOR THE DETECTION OF CIRCULATING TUMOR CELLS USING IMMUNOMAGNETIC BEADS AND THE POLYMERASE CHAIN REACTION" CANCER RESEARCH, AMERICAN ASSOCIATION FOR CANCER RESEARCH, BALTIMORE, MD, US, Bd. 53, 1. August 1993 (1993-08-01), Seiten 3455-3458, XP000605489 ISSN: 0008-5472
- ZHONG X Y ET AL: "EVALUATING GA733-2 MRNA AS A MARKER FOR THE DETECTION OF MICROMETASTATIC BREAST CANCER IN PERIPHERAL BLOOD AND BONE MARROW" ARCHIVES OF GYNECOLOGY AND OBSTETRICS, SPRINGER VERLAG, BERLIN, DE, Bd. 263, Nr. 1/2, 1999, Seiten 2-6, XP000929386 ISSN: 0932-0067
- BURCHILL S A ET AL: "DETECTION OF EPITHELIAL CANCER CELLS IN PERIPHERAL BLOOD BY REVERSETRANSCRIPTASE-POLYMERASE CHAIN REACTION" BRITISH JOURNAL OF CANCER, LONDON, GB, Bd. 71, Nr. 2, 1995, Seiten 278-281, XP000562468 ISSN: 0007-0920
- NOTTERMAN DANIEL A ET AL: "Transcriptional gene expression profiles of colorectal adenoma, adenocarcinoma, and normal tissue examined by oligonucleotide arrays." CANCER RESEARCH, Bd. 61, Nr. 7, 1. April 2001 (2001-04-01), Seiten 3124-3130, XP002250499 ISSN: 0008-5472
- REN-HEIDENREICH L ET AL: "SPECIFIC TARGETING OF EGP-2+ TUMOR CELLS BY PRIMARY LYMPHOCYTES MODIFIED WITH CHIMERIC T CELL RECEPTORS" HUMAN GENE THERAPY, XX, XX, Bd. 11, Nr. 1, 1. Januar 2000 (2000-01-01), Seiten 9-19, XP000944357 ISSN: 1043-0342
- ITO M ET AL: "EXPRESSION OF SEVERAL GROWTH FACTORS AND THEIR RECEPTOR GENES IN HUMAN COLON CARCINOMAS" VIRCHOWS ARCHIV B CELL PATHOLOGY INCLUDING MOLECULAR PATHOLOGY, Bd. 59, Nr. 3, 1990, Seiten 173-178, XP009015191 ISSN: 0340-6075
- FUJIWARA Y ET AL: "ASSESSMENT OF STANNIOCALCIN-1 MRNA AS A MOLECULAR MARKER FOR MICROMETASTASES OF VARIOUS HUMAN CANCERS" INTERNATIONAL JOURNAL OF ONCOLOGY, EDITORIAL ACADEMY OF THE INTERNATIONAL JOURNAL OF ONCOLOGY,, GR, Bd. 16, April 2000 (2000-04), Seiten 799-804, XP002938551 ISSN: 1019-6439

## Beschreibung

Die vorliegende Erfindung bezieht sich auf ein Verfahren und ein Kit zur Diagnostik oder Behandlungskontrolle von Darmkrebs bei einem Menschen

Bei der Krebsnachsorge ist es von Relevanz, ein Rezidiv maligner Tumore anhand des Auftretens metastasierender Tumorzellen im Blut frühzeitig nachweisen zu können. Bei den derzeit angewandten Untersuchungsmethoden werden bei Krebspatienten sogenannte "Tumormarker" auf Proteinebene (immunologisch bzw. enzymatisch) quantitativ im Blut oder in anderen Körperflüssigkeiten ermittelt.

Diese Nachweisverfahren sind für die Tumordiagnostik bzw. Behandlungskontrolle/Nachsorge nur bedingt geeignet, da erhöhte Tumormarkerwerte auch durch nicht-Tumorerkrankungen (z.B. Entzündungen des Magen-DarmTraktes, Leberzirrhose, Virusinfekte), starkes Rauchen oder durch eine Schwangerschaft hervorgerufen werden können.

Die Tumorprogression nach der Resektion eines kolorektalen Primärtumors ist in erster Linie auf residuale Tumorzellen zurückzuführen. Diese Zellen werden prä- oder intraoperativ aus dem primären Tumor abgelöst und erhalten die Möglichkeit zur Verstreuung im ganzen Organismus.

Neben der Ersterkennung eines kolorektalen Karzinoms ist daher insbesondere der frühest mögliche Nachweis metastasierender Zellen für eine erfolgreiche Behandlung von entscheidender Bedeutung. Ebenso kann im klinischen Stadium I ein definitiver Negativnachweis hilfreich sein, wenn zu entscheiden ist, ob der Patient mit einer Chemotherapie oder einer Operation belastet werden muß.

Die derzeit verwendeten diagnostischen Methoden sind ungenau, wenn es um die Beurteilung der malignen Potenz von Resttumoren nach durchgeführter Chemotherapie in den metastasierenden Stadien geht. Einige klinische Studien weisen auf eine prognostische Bedeutung von disseminierten Tumorzellen hin. Dennoch sind zahlreiche methodische Aspekte kritisch und bisher nicht hinreichend standardisiert. Somit müssen weiterhin Nachweise für eine okkulte oder restliche Metastasierung gefunden werden, die eine rechtzeitige Einordnung in die vielfältigen primär kurativen therapeutischen Optionen zulassen.

Dem Bestreben nach Verbesserung der Heilungschancen wird heute einerseits über Suche und Verwendung neuer Tumormarker, andererseits über Sensitivitätssteigerung bei den verwendeten Methoden nachgekommen.

Die DE 197 36 691 A1 offenbart ein Verfahren zur Charakterisierung und Identifizierung disseminierter und metastasierter Krebszellen. Dabei wird eine Körperflüssigkeit auf wenigstens ein krebsspezifisches Gen und wenigstens ein krebsassoziiertes Gen untersucht. Diese Druckschrift schlägt in allgemeiner Form den Einsatz von Multiparameterexpressionsanalysen vor.

Die WO 94/07139 offenbart ebenfalls ein Verfahren, um Zielzellen in Zellsuspensionen, wie beispielsweise Blutproben, zu erfassen. Hierzu werden die Zielzellen immunologisch aus der Zellsuspension abgetrennt und anschließend gezählt.

Die WO 99/44064 A1 offenbart ein Verfahren zur differenziellen Erfassung von Primärtumorzellen und metastasierenden Zellen in einer Blutprobe. Hierzu werden die metastasischen Zellen erfasst, indem mittels Detektormolekülen die Expression des epithelialen Zellantigens und des IL-2Ra-Moleküls erfasst wird. Die Zielzellen zeichnen sich dann durch eine Expression beider Moleküle aus.

Smith et al., The Lancet, Bd. 338, Seiten 1227-1229 (16.11.91) offenbart ein Verfahren, um Tumorzellen in einer Blutprobe zu erfassen. Hierzu wird zuerst aus einer Blutprobe die RNA in cDNA umgeschrieben und anschließend mittels Polymerasekettenreaktion analysiert. Eine Abtrennung der Tumorzellen mittels eines immunologischen Verfahrens vor Analyse der mRNA-Expression findet hier nicht statt.

Ghossein et al., Clin. Cancer Res., Bd. 5, Nr. 8, Seiten 1950-1960 (08.99) offenbart ebenfalls ein Verfahren zur Erfassung von zirkulierenden Tumorzellen und Mirkometastasen in einer Blutprobe. Dieses basiert wie bei vorhergehend beschriebenen Druckschrift auf der Amplifikation von in cDNA umgeschriebener mRNA.

Hardingham et al., Cancer Res., Bd. 53, Seiten 3455-3458 (01.08.93) zeigt die Abtrennung von Tumorzellen mittels eines immunologischen Nachweises, bei dem ein ausgesuchter Antikörper an magnetische Beads gebunden wird. Die so abgetrennten Zellen werden anschließend auf eine Punktmutation in dem K-ras-Gen, das in den untersuchten Tumorzellen vorhanden ist, analysiert.

Zhong et al., Arch. Gynecol. Obstet., Bd. 263, Nr. 1/2, Seiten 2-6, (1999) zeigt die Verwendung des mRNA-Markers GA733.2 zur Erfassung von zirkulierenden Brusttumorzellen in peripherem Blut.

Notterman et al., Cancer Res., Bd. 61, Nr. 7, Seiten 3124-3130 (01.04.01) offenbart Genexpressionsprofile für verschiedene Tumorarten. Diese werden durch Analyse der mRNA in den Tumorzellen ermittelt.

Burchill et al., Br. J. Cancer, Bd. 71, Nr. 2, Seiten 278-281 (1995) zeigt in einer weiteren Arbeit die Erfassung von epithelialen Tumorzellen in Proben mit peripherem Blut. Hierzu wird über eine Reverse-Transkriptase-Polymerasekettenreaktion die Expression der Gene CK8, CK19 und CK20 analysiert.

Ren-Heidenreich et al., Hum. Gene Ther., Bd. 11, Nr. 1, Seiten 9-19 (01.01.00) befasst sich mit dem EGP-2 epithelialen Glycoprotein, das in verschiedenen humanen Tumoren überexprimiert wird.

Ito et al., Virchows Archiv B Cell Pathol., Bd. 59, Nr. 3, Seiten 173-178 (1990) beschäftigt sich mit verschiedenen Tumormarkern, beispielsweise EGF-R und PDGF-β sowie deren Expressionsprofil in Tumorzellen. Hierzu wird die mRNA in diesen Zellen analysiert.

Die WO 97/37226 A1 zeigt ein Verfahren zur Gewinnung von colorektalen epithelialen Zellen aus Stuhl. Hierzu werden diese über Antikörper markiert und anschließend absepariert. Als Zielepitope werden das MUC-1-Antigen bzw. das P53-Antigen vorgeschlagen.

Fujiwara et al., Int. J. Oncol., Bd. 16, Seiten 199-804, April 2000 beschäftigt sich mit Stanniocalcin als mRNA-Tumormarker für Mikrometastasen verschiedener humaner Tumore. Hierzu wird die Expression von Stanniocalcin mittels RT-PCR ermittelt.

Aufgabe der vorliegenden Erfindung ist es, ein Verfahren und ein Kit zur Verfügung zu stellen, mit dem auf einfache, sichere und wiederholbare Weise eine Diagnostik oder Behandlungskontrolle von Darmkrebs möglich ist.

Diese Aufgabe wird durch das Verfahren nach Patentanspruch 1 sowie das Kit nach Patentanspruch 27 gelöst. Vorteilhafte Weiterbildungen des Verfahrens und des Kits werden in den jeweiligen abhängigen Ansprüchen gegeben.

Erfindungsgemäß wird mittels des erfindungsgemäßen Kits bzw. des erfindungsgemäßen Verfahrens in einer Blutprobe eines Menschen das Vorhandensein oder Fehlen von mRNS mindestens der Tumormarkerproteine EGF-R, CEA und GA733.2 erfaßt.

Da im Blut Gesunder die RNAs der beschriebenen Marker normalerweise nicht exprimiert vorliegen, zeigt sich eine direkte Korrelation zwischen einem positiven RT-PCR-Nachweis dieser Tumormarker und zirkulierenden Tumorzellen im Blut, die zu einer Metastasierung führen können.

Da einzelne Marker in therapieabhängiger Weise unterschiedlich exprimiert werden können, ist es vorteilhaft, eine Kombination von drei, vier oder mehr Tumormarkern zu untersuchen, um alle im Blut zirkulierende Tumorzellen zu erfassen. Hierdurch lassen sich Tumorzellen auch dann erkennen, wenn die Expression eines bestimmten Markers bei einem Patienten bzw. in einem Krankheitsstadium relativ gering ist, was sonst zu einem vermeintlich negativen Ergebnis führen könnte. Die Verwendung weiterer Marker stößt jedoch meist deswegen auf Grenzen, wenn mononukleäre Blutzellen eine Hintergrundexpression ("illegitime Transkription") aufweisen, die eine exakte Analyse behindern.

Für die Erkennung von Darmtumorzellen wird daher erfindungsgemäß eine der folgenden Kombinationen aus Markern als besonders vorteilhafte Markerkombination vorgeschlagen, mit denen die genannten Probleme dennoch vermieden werden können, vorgeschlagen:
GA733.2, CEA und EGF-R;
CK20, EGFR, GA733.2, CEA und Stanniocalcin; oder EGFR, CEA und GA733.2;
GA733.2, PDGF-β, CEA und EGF-R.

Im folgenden werden die entsprechenden Marker erläutert.

| **Gen bzw. Genprodukt** | **Gen** | **Alernativ- bezeichn..** |
|---|---|---|
| Human carninoma-associated antigen GA733-2 gene | GA733-2 | GA733.2 |
| Human epidermal growth factor receptor (EGFR) gene | EGFR | EGFR |
| Human carcinoembryonic antigen (CEA) gene | CEA | CEA |
| Homo sapiens gene for cytokeratin 20 | CK20 | CK20 |
| Homo sapiens stanniocalcin 1 (STC1) gene | Stanniocalcin 1 (STC1) | Stanniocalcin |
| Platelet derived growth factor-β | PDGF-β | PDGF-β |

Bei der Anwendung von RT-PCR-Systemen zum Nachweis von Tumorzellen ist aufgrund der sehr hohen Amplifikationsrate die Spezifität ein kritischer Punkt. Geringste Kontaminationen, etwa durch Fremd-RNA oder illegitimer Transkription, können hierbei das Ergebnis verfälschen.

Durch die Verwendung der Immunzytochemie mit monoklonalen Antikörpern gegen Tumorzellantigene kann eine Steigerung der Spezifität durch Anreicherung der Tumorzellen gegenüber Blutzellen und gleichzeitig eine Steigerung der Sensitivität des Tumorzell-Nachweises erzielt werden (Nachweisquote von 1 Tumorzelle auf 10⁷ mononukleären Blutzellen). Die Tumorzellen werden dabei mittels spezifischer Antikörper bzw. einer Antikörpermischung von mononukleären Blutzellen getrennt. Die Trennung kann mittels Magnetpartikel (Dynal), an die die Antikörper gebunden werden, erfolgen. Dies wird im folgenden detaillierter beschrieben.

Eukaryontische Zellen tragen eine Vielzahl unterschiedlicher Moleküle an ihrer Zelloberfläche. Entsprechend des Ursprungs und der Funktion der einzelnen Zelle unterscheidet sich die Kombination der exprimierten Oberflächenmoleküle, so daß zelltypspezifische Muster entstehen. Zur Erkennung dieser zelltyp-spezifischen Muster werden Antikörper genutzt. Antikörper binden mit hoher Spezifität an ihr Antigen, hier an ausgewählte Oberflächenmoleküle. Diese Eigenschaft wird genutzt, um Zellen mittels spezifischer Antikörperbindung anhand ihrer Zelltyp-spezifischen Muster zu erkennen und voneinander zu unterscheiden.

Die Expression spezieller Oberflächenproteine unterscheidet Tumorzellen von nichttransformierten Zellen dieses Zelltyps. Da sich dieses spezielle Muster der Oberflächen-Antigene bei Tumorzellen auch von den Blutzell-typischen Mustern unterscheidet, können Tumorzellen im Blut unterschieden werden. Um Tumorzellen zu identifizieren, werden Antikörper, die diese speziellen Oberflächenproteine spezifisch erkennen, als Werkzeuge genutzt. Die spezifische Antikörperbindung wird für verschiedene Analyse- und Separations-Methoden nutzbar gemacht.

Aufgrund der intensiven Bindung von speziell dafür selektionierten Immunglobulinen ist neben der Erkennung von Zellen über deren Oberflächenepitope auch eine Separierung der erkannten Zellen von nicht erkannten möglich.
1. Trennprinzip beruhend auf Flüssigphase; z. B. Durchflußzytometrie:
   Für die durchflußzytometrische Analyse werden Antikörper mit Fluoreszenzfarbstoffen gekoppelt. Vereinzelte Zellen werden in einem konstanten Flüssigkeitsstrom einzeln an einer Lichtquelle (Laser) vorbeigeleitet. Bei Beleuchtung der Zellen werden die an den Antikörpern gebundenen Fluoreszenzfarbstoffe angeregt und strahlen Licht bestimmter Wellenlängen ab. Das abgestrahlte Licht wird detektiert und das gemessene Signal digitalisiert gespeichert. Das Lichtsignal kann einzelnen Zellen zugeordnet werden. Die Antikörper-markierte Zelle wird so erkannt und kann nun von anderen Zellen getrennt werden. Zur Trennung werden die Zellen in kleinsten Tropfen vereinzelt. Nach Erkennung der Antikörper-markierten Zelle wird der entsprechende Tropfen in ein Auffangbehältnis gelenkt.
2. Trennprinzip beruhend auf Festphase; z. B. magnetische Separation:
   Für die magnetische Separation werden Antikörper an pseudomagnetische Partikel gekoppelt. Nach Einbringen der pseudomagnetischen Partikel in ein Magnetfeld wandern die Partikel im magnetischen Feld. Bei der Bewegung in diesem magnetischen Feld werden Zellen, an die diese gekoppelten Antikörper gebunden sind, mitgerissen und von anderen Zellen getrennt.
   Zur Tumorzellerkennung mittels Magnetpartikel werden folglich an pseudomagnetische Partikel, die eine definierte Anzahl an chemisch aktivierten Stellen auf ihrer Oberfläche besitzen, Antikörper kovalent gekoppelt. Über die Spezifität der Antikörper wird die Spezifität der Trennung bestimmt. Eine Tumorzellen enthaltende Blutprobe wird mit Antikörper-gekoppelte Magnetpartikel versetzt; dann werden Partikel und Blut relativ zueinander bewegt. Jene (Tumor-)Zellen, die von den Festphase-gebundenen Antikörpern erkannt und damit fest gebunden werden, folgen der Bewegung der Partikel. Hierdurch ist es möglich, bei Anlegen eines magnetischen Feldes, die Partikel mit den daran gebundenen Zellen aus dem Blut herauszuziehen (z.B. an die Wand des Trenngefäßes). Das auf diese Weise Tumorzellen-depletierte Blut kann gegen andere Lösungen ausgetauscht werden, wobei die über Magnetpartikel separierten Zellen bis zum Abschalten/Entfernen des Magnetfeldes vor Ort verbleiben und für weitere Anwendungen zur Verfügung stehen.
   Vorteilhafterweise können für die Erkennung der Tumorzellen spezifische Antikörper-Mischungen verwendet werden, die entweder allgemein auf Tumorzellen oder auch spezifisch für Darmtumorzellen-Erkennung optimiert sind. Beispielsweise eignet sich zur Erkennung von Tumorzellen im Blut eine Kombination der Antikörper MOC-31 und Ber-EP4.
   Derartige Antikörpermischungen zeigen im Vergleich zu den jeweils separat eingesetzten Antikörpern bei der Zellerkennung und Zelltrennung unabhängig von der angewandten Methode eine erhöhte Sensitivität.

Im folgenden sollen einige Beispiele erfindungsgemäßer Nachweise für Darmtumorzellen in Blutproben beschrieben werden.

Es zeigen:
- Fig. 1: einen Nachweis von PCR-Produkten mit Elektrophorese;
- Fig. 2: einen weiteren Nachweis von PCR-Produkten mit Elektrophorese;
- Figuren 3A bis 3D: einem Tumormarkernachweis mittels Light Cycler;
- Fig. 4: den Nachweis einer Zelltrennung mittels Antikörper-markierter Magnetpartikel,
- Fign. 5 bis 7: den Nachweis von Darmtumorzellen durch verschiedene Markerkombinationen.

In einem ersten Beispiel erfolgte eine RNA-Aufarbeitung aus 1 ml EDTA-Vollblut mit dem QIAamp RNA Blood Mini Kit (Fa. Qiagen, Hilden). Kontaminationen mit genomischer DNA wurden durch einen zusätzlichen DNA-Verdau auf der Säule mittels RNase-free DNase Set, Fa. Qiagen, Hilden) vermieden.

Die RNA-Aufarbeitung aus 1 ml EDTA-Vollblut wurde fotometrisch über den Quotienten 260 : 280 nm verifiziert. Zur Qualitäts- und Quantitätsbestimmung kann dabei 1 µl des Ansatzes durch elektrophoretische Trennung auf einem RNA 6000 Chip über den Agilent Bioanalyzer 2100 analysiert werden.

Die isolierte RNA wurde in einem entsprechenden Volumen zusammen mit Oligo(dT)15-Primern (Fa. Promega, Mannheim) für 5 Minuten bei 65°C denaturiert und anschließend direkt auf Eis inkubiert. Die cDNA-Synthese erfolgte mittels des Sensiscript^{™} Reverse Transkriptase Kit, (Fa. Qiagen, Hilden) in einem 20 µl Reaktionsansatz gemäß Tabelle 1 bei 37°C für 1 Stunde mit nachfolgender Inaktivierung der reversen Transkriptase für 5 Minuten bei 95°C und anschließender Abkühlung auf Eis.

**Tabelle 1: Komponenten der cDNA-Synthese**

| Komponenten | Volumen | Endkonzentration |
|---|---|---|
| RNA | X µl | 5 ng/ µl |
| 10xRT-Puffer | 2 µl | 1x |
| dNTP-Mix (je 5 mM) | 2 µl | jeweils 0,5 mM |
| Oligo (dT)-Primer (10 µM) | 2 µl | 1 µM |
| RNase-Inhibitor | 1 µl | 0,5 Units/ µl |
| Reverse Transkriptase | 1 µl | 4 U |
| RNase-freies Wasser | ad 20 µl | |

Mit der so erzeugten cDNA wurde für jeden der gewählten Tumormarker Stanniocalcin, EGF-R, CK 20, CEA sowie als interne Kontrolle für β-Aktin eine Multiplex-PCR durchgeführt. Der PCR-Ansatz ist in der folgenden Tabelle 2 dargestellt.

**Tabelle 2: PCR-Ansatz**

| Komponenten | Volumen | Endkonzentration |
|---|---|---|
| cDNA | 8 µl | |
| 10x PCR.Puffer* | 5 µl | 1x |
| dNTP-Mix | 1 µl | jeweils 200 µM |
| Primer | (s. Tabelle 3) | |
| DMSO-Zusatz *** | 1,0 µl | |
| Taq-DNA Polymerase ** | 0,5 µl | 2,5 U |
| H₂O | ad 50 µl | |

| | | |
|---|---|---|
| (*enthält 15 mM MgCl₂; für Stanniocalcin 20 mM MgCl_{2;} **HotStarTaq^{™} DNA Polymerase; Fa. Qiagen, Hilden; ***DMSO-Zusatz bei Stanniocalcin). | | |

Für jeden Tumormarker wurde dabei ein Primerpaar eingesetzt, das aus der nachfolgenden Tabelle 3 hervorgeht.

**Tabelle 3: Liste der PCR-Primer**

| Primername | Sequenz 5'→ 3' | PCR-Produkt |
|---|---|---|
| Tumormarker | | |
| Stanniocalcin sense | AACCCATGAGGCGGAGCAGAATGA | 254 bp |
| Stanniocalcin antisense | CGTTGGCGATGCATTTTAAGCTCT | |
| EGF-R sense | AGTCGGGCTCTGGAGGAAAAGAAA | 163 bp |
| EGF-R antisense | GATCATAATTCCTCTGCACATAGG | |
| CK20 sense | ATCTCCAAGGCCTGAATAAGGTCT | 336 bp |
| CK20 antisense | CCTCAGTTCCTTTTAATTCTTCAGT | |
| CEA sense | AGAAATGACGCAAGAGCCTATGTA | 231 bp |
| CEA antisense | AACTTGTGTGTGTTGCTGCGGTAT | |
| GA733.2 sense | AATCGTCAATGCCAGTGTACTTCA | 395bp |
| GA733.2 antisense | TAACGCGTTGTGATCTCCTTCTGA | |
| PDGF-β sense | TCTCTCTGCTGCTACCTGCGTCTG | |
| PDGF-β antisense | GTTGGCGTTGGTGCGGTCTATGAG | |

| interne Kontrolle | | |
|---|---|---|
| β-Aktin sense | CTGGAGAAGAGCTACGAGCTGCCT | 116 bp |
| β-Aktin antisense | ACAGGACTCCATGCCCAGGAAGGA | |

Die für die Tumormarkereinzelnachweise eingesetzten Primerkombinationen und -mengen sind in der folgenden Tabelle 4 aufgeführt.

**Tabelle 4: Auflistung der Primermengen und Primerkombinationen**

| Marker | Stanniocalcin | EGF-R | CK20 | CEA |
|---|---|---|---|---|
| Primer | | | | |
| Stanniocalcin sense | 25 pmol | | | |
| Stanniocalcin antisense | 25 pmol | | | |
| EGF-R sense | | 25 pmol | | |
| EGF-R antisense | | 25 pmol | | |
| CK20 sense | | | 25 pmol | |
| CK20 antisense | | | 25 pmol | |
| CEA sense | | | | 25 pmol |
| CEA antisense | | | | 25 pmol |
| β-Aktin sense | 1 pmol | 1 pmol | 1 pmol | 1 pmol |
| β-Aktin anti-sense | 1 pmol | 1 pmol | 1 pmol | 1 pmol |

Die PCR wurde unter den in Tabelle 5 angegebenen PCR-Bedingungen und mit den in Tabelle 6 angegebenen Marker-spezifischen Schmelz-Temperaturen und Zyklenzahlen durchgeführt.

**Tabelle 5: PCR-Bedingungen**

| | | | | |
|---|---|---|---|---|
| Vorabdenaturierung | | 95°C | 15 min | |
| Zyklus | | | | |
| | 1. Denaturierung | 94°C | 1min | |
| | 2. Annealing | X°C | 1 min | (s. Tabelle 6) |
| | 3. Extension | 72°C | 1 min | |
| Finale Extension | | 72°C | 10 min | |
| | | 4°C | Pause | |

**Tabelle 6: Marker-spezifische Annealing-Temperatur und Zyklenzahl**

| Marker | Stanniocalcin | EGF-R | CK20 | CEA |
|---|---|---|---|---|
| Annealing-Temperatur | 58°C | 64°C | 58°C | 60°C |
| Zyklenzahl | 45 | 40 | 35 | 40 |

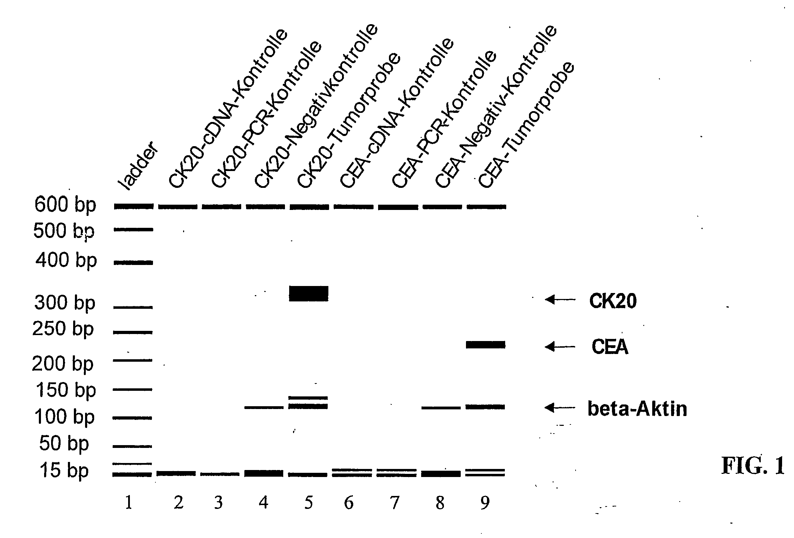

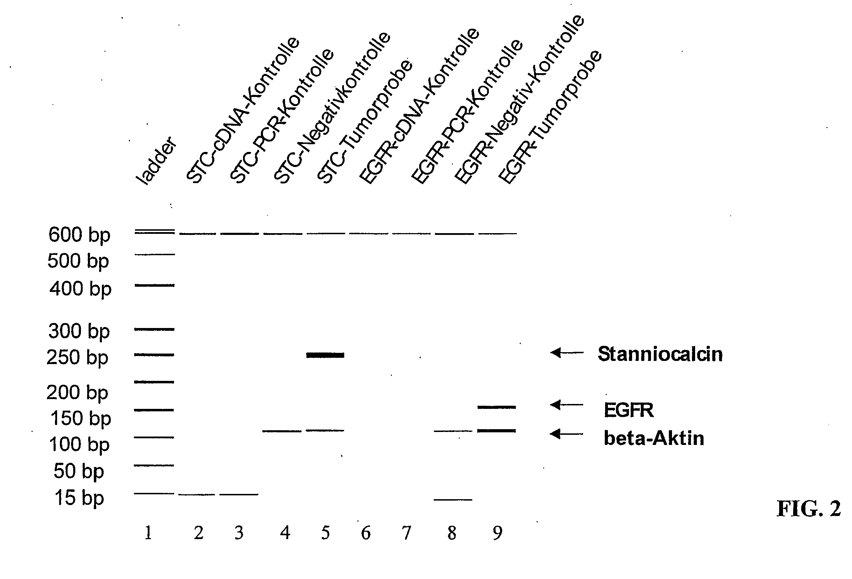

1 µl des so erzeugten PCR-Produktes wurde in einem Agilent Bioanalyzer 2100 auf einem DNA-Chip (500) aufgetrennt und das Trennergebnis elektronisch dokumentiert. Die Ergebnisse zeigen die Figuren 1 und 2. In diesen Figuren zeigt jeweils Spur 1 eine 100 kb-Leiter sowie die Spuren 2-9 die Ergebnisse der entsprechenden Proben. Wie zu erkennen ist, zeigt Spur 5 ein PCR-Produkt für den Tumormarker CK20 und Spur 9 ein PCR-Produkt für den Tumormarker CEA, während sämtliche Proben mit biologischem Material (Spuren 4, 5, 8, 9) PCR-Produkte für die interne Kontrolle β-Aktin enthalten.

In Fig. 2 zeigen die Spuren 5 und 9 PCR-Produkte der Marker Stanniocalcin bzw. EGF-R, während wiederum die biologisches Material enthaltenden Proben gemäß Spuren 4, 5, 8, 9 PCR-Produkte von β-Aktin als internem Marker enthalten.

Die Spuren 2, 3, 6, 7 enthalten kein biologisches Material, so daß dort auch keine entsprechenden PCR-Produkte auftreten. Die sogenannte cDNA-Kontrolle ist ein Ansatz ganz ohne RNA, die sogenannte PCR-Kontrolle ist ein Ansatz ohne cDNA und die Negativ-Kontrolle ist ein Ansatz mit RNA einer gesunden Kontrollperson in Fig. 1 und in Fig. 2. CEA steht für Carcinoembryonic Antigen, CK20 für Cytokeratin 20, STC für Stanniocalcin und EGF-R für Epidermal Growth Factor Receptor in den Figuren 1 und 2.

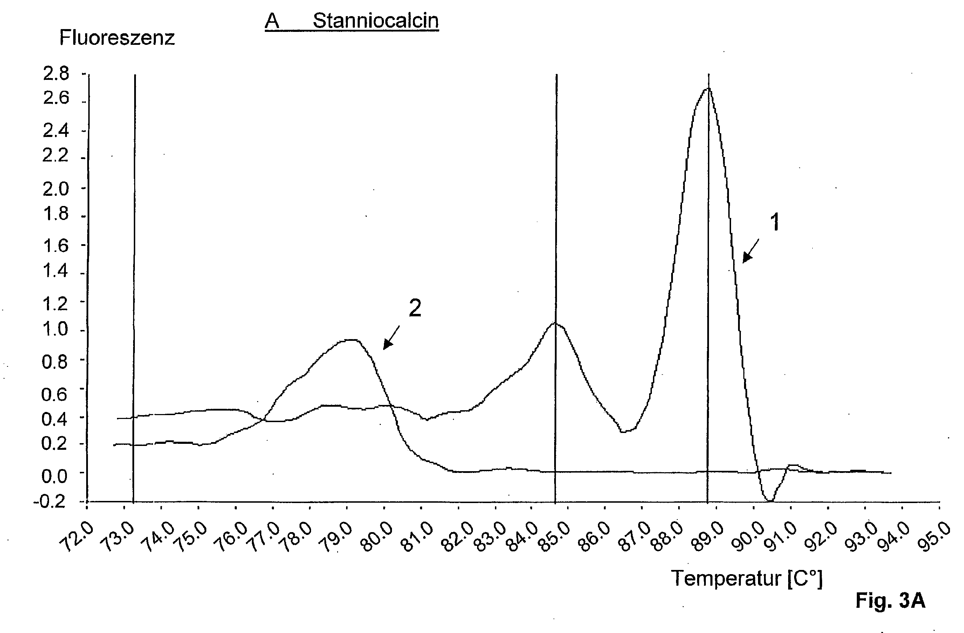

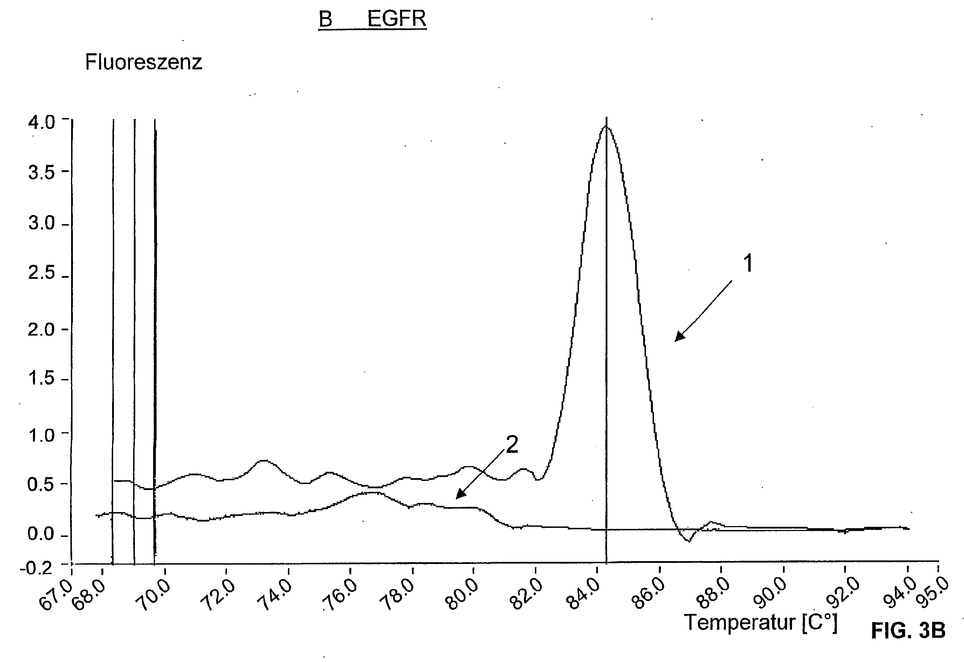

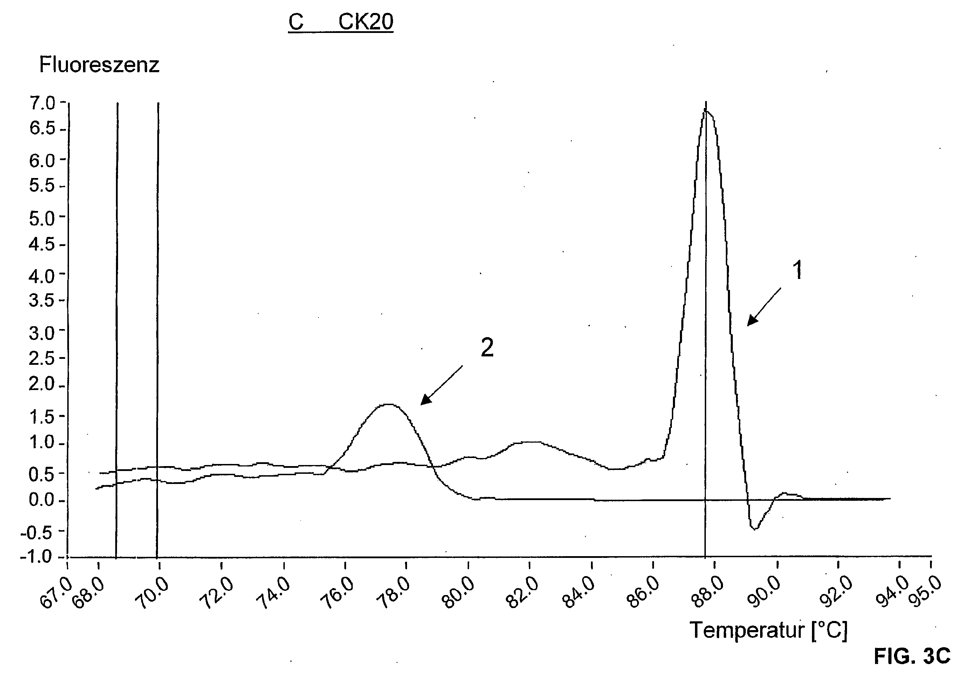

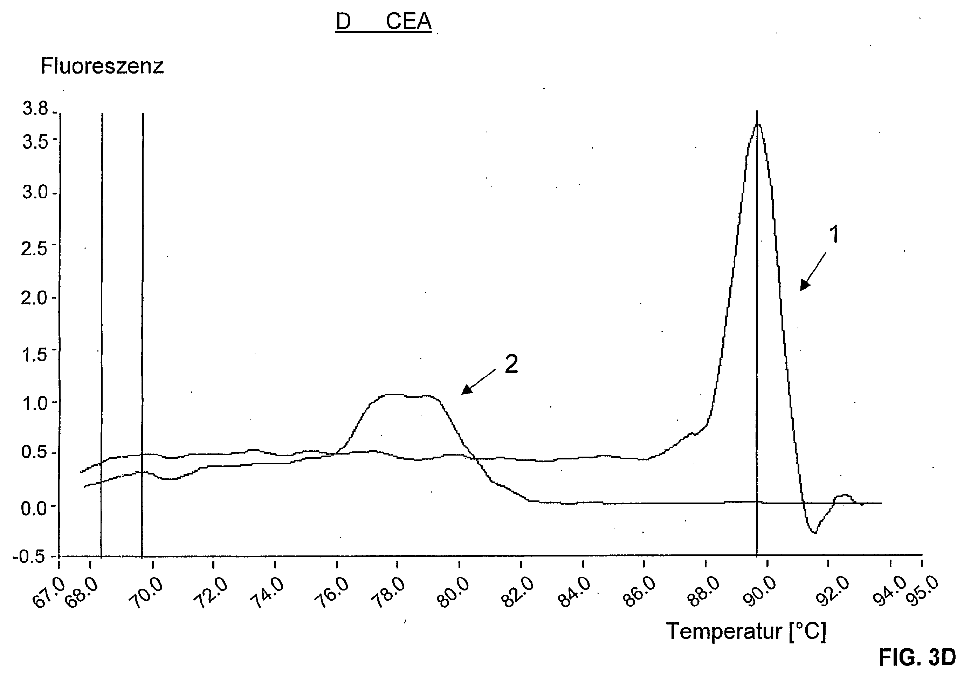

Fig. 3 zeigt die alternative Analyse mittels Fluoreszens-basierender Echtzeit-PCR mittels interkallierender Fluoreszenzfarbstoffe.

Dieser Tumormarkernachweis kann alternativ zur Block-PCR auch mittels Light Cycler (Fa. Roche, Basel) erfolgen.

Die reverse Transkription der mRNA erfolgt wie oben beschrieben. Anschliessend wurde die PCR mit dem Light Cycler-DNA Master Sybr Green I Kit (Fa. Roche, Basel) nach Herstellerangaben unter den für jeden Tumormarker optimierten Bedingungen durchgeführt. Als Primer wurden dabei die in Tabelle 3 angegebenen Oligonukleotide verwendet. Tabelle 7 und Tabelle 8 zeigen den Ansatz für die PCR bzw. die PCR-Bedingungen im Light Cycler.

**Tabelle 7: PCR-Ansatz LightCycler**

| Tumormarker | Stannio-calcin | EGF-R | CK20 | CEA |
|---|---|---|---|---|
| Komponenten | | | | |
| CDNA | 3,0 µl | 3,0 µl | 3,0 µl | 2,0 µl |
| MgCl₂ | 3,0 mM | 3;5 mM | 3,5 mM | 2,5 mM |
| Primer | je 0,5 µM | je 0,5 µM | je 0,5 µM | je 0,5 µ |
| Light Cycler-DNA Master Sybr Green | 2 µl | 2 µl | 2 µl | 2 µl |
| DMSO | 1 µl | - | - | - |
| H₂O | ad 20 µl | ad 20 µl | ad 20 µl | ad 20 |

**Tabelle 8: PCR-Bedingungen LightCycler**

| | Stanniocalcin | EGFR | CK20 | CEA |
|---|---|---|---|---|
| Denaturierung | 95°C, 30 Sek | 95°C, | 95°C, | 95°C, |
| | 20°C/Sek | 30 Sek | 30 Sek | 30 Sek |
| | | 20°C/Sek | 20°C/Sek | 20°C/Sek |
| Amplifikation | 95°C, 5 Sek, | 95°C, | 95°C, | 95°C, |
| | 20°C/Sek | 5 Sek, | 5 Sek, | 5 Sek, |
| | | 20°C/Sek | 20°C/Sek | 20°C/Sek |
| | 67°C, 10 Sek, | 60°C, | 58°C | 60°C, |
| | 20°C/Sek | 10 Sek, | 10 Sek, | 10 Sek, |
| | | 20°C/ | 20°C/Sek | 20°C/Sek |
| | 73°C, 15 Sek, | 73°C, | 73°C, | 73°C, |
| | 5°C/Sek | 15 Sek, | 20 Sek, | 20 Sek, |
| | | 5°C/Sek | 5°C/Sek | 5°C/Sek |
| Melting Curve | 95°C, 0 Sek, | 95°C, | 95°C, | 95°C, |
| | 20°C/Sek | 0 Sek, | 0 Sek, | 0 Sek, |
| | | 20°C/Sek | 20°C/Sek | 20°C/Sek |
| | 70°C, 20 Sek, | 65°C, | 65°C, | 65°C, |
| | 20°C/Sek | 20 Sek, | 15 Sek, | 15 Sek, |
| | | 20°C/Sek | 20°C/Sek | 20°C/Sek |
| | 95°C, 0 Sek, | 95°C, | 95°C, | 95°C, |
| | 0,1°C/Sek | 0 Sek, | 0 Sek, | 0 Sek, |
| | | 0,1°C/Sek | 0,1°C/Sek | 0,1°C/Sek |
| Cooling | 30°C, 30 Sek | 30°C, | 30°C, | 30°C, |
| | 20°C/Sek | 30 Sek | 30 Sek | 30 Sek |
| | | 20°C/Sek | 20°C/Sek | 20°C/Sek |

Das Ergebnis dieser PCR und Auswertung durch Light Cycler-Technologie ist in den Figuren 3A bis 3D dargestellt.

In sämtlichen Figuren 3A bis 3D ist die Kontrollkurve mit 2 bezeichnet, während die Kurve, die für die Probe aufgenommen wurde, mit 1 bezeichnet ist.

Bei dieser Analyse wird die Schmelzkurve der PCR-Produkte durch Sybr Green I Detektion analysiert. Die jeweilige Graphik der Figuren 3A bis 3D stellt dabei die gemessene Fluoreszenz in Abhängigkeit von der Temperatur dar. Die in den Kontrollansätzen auftretenden Fluoreszenzpeaks sind auf Primerdimere zurückzuführen.

Fig. 3A stellt dabei die Schmelzkurvenanalyse des Stanniocalcin-PCR-Produktes dar. Der Schmelzpunkt des Hauptproduktes liegt bei 88,7°C und der Schmelzpunkt des Nebenproduktes bei 84,6°C. Derartige Fluoreszenzpeaks sind bei der Kontrollprobe nicht zu erkennen.

Fig. 3B zeigt die Schmelzkurvenanalyse des EGFR-PCR-Produktes mit einem Schmelzpunkt bei 84,3°C.

Fig. 3C zeigt eine Schmelzkurvenanalyse des CK20-PCR-Produktes mit einem Schmelzpunkt bei 87,6°C.

Fig. 3D zeigt die Schmelzkurvenanalyse des CEA-PCR-Produktes mit einem Schmelzpunkt bei 89,7°C.

Alternativ zu den hier dargestellten Methoden können selbstverständlich auch herkömmliche Analysemethoden wie Agarose-Gelelektrophorese angewandt werden, bei denen beispielsweise 25 µl des oben dargestellten PCR-Produktes über ein 2,5%iges Agarosegel aufgetrennt werden und die DNS-Banden anschließend mit Ethidiumbromid angefärbt und sichtbar gemacht werden. Die Dokumentation kann beispielsweise mit Hilfe des DUO Store Systems der Fa. Intas durchgeführt werden.

Auch eine Fragmentanalyse mittels ABI Prism 310 Genetic Analyser (Fa. PE Applied Biosystems, Weiterstadt) kann zur Auswertung verwendet werden. Hierzu wird eine PCR mit fluoreszenzmarkierten Primern durchgeführt und anschließend beispielsweise jeweils 1 µl des jeweiligen PCR-Produktes in einer Verdünnung von 1 : 50 eingesetzt.

Als weiterer Nachweis ist ein Nachweis mittels sequenzspezifischer fluoreszenzmarkierter Hybridisierungsproben möglich, die nach jedem Zyklus der PCR die Produktentwicklung verfolgen lassen. Anhand spezieller Standards kann dann auch ein Rückschluß auf die Menge an Ausgangs-RNS gezogen werden.

Zentral für die Qualität der als Nachweisbasis isolierten RNS und der daraus synthesisierten cDNS ist die Anreicherung der hierfür verwendeten Zellfraktion aus der verwendeten Blutprobe. Hierfür stehen vier verschiedene Methoden zur Verfügung:
a) Anreicherung durch wiederholte Zentrifugation nach Erythrozytenlyse:
   1 ml EDTA-Blut werden nach Zugabe von 5 Volumina Erythrozyten-Lysepuffer ("QIAmp Blood Kit", Qiagen; Hilden) für 20 Minuten auf Eis lysiert. Nach Abnehmen des Plasmas/Lysates von den pelletierten Zellen und Resuspension erfolgt eine erneute Zentrifugation bei 3000 x g für 20 Minuten. Nach Abnehmen des Überstandes steht die pelletierte Leukozytenfraktion für die RNA-Präparation zur Verfügung.
b) Anreicherung durch Dichtegradienten-Zentrifugation:
   Mittels eines über Zentrifugation erzeugten Dichtegradienten lassen sich Zellen unterschiedlicher mittlerer Volumendichte voneinander trennen. Mononukleare Blutzellen werden mittels eines Ficoll-Hypaque-Gradienten (Pharmacia, Uppsala, Schweden) separiert und anschließend zweifach mit PBS/1% FCS gewaschen.
c) Anreicherung von Tumorzellen durch FACS-Durchflußzytometrie:
   Die mononukleären Zellen aus der unter b) angereicherten Fraktion werden mit fluoreszenzmarkierten mononuklearen Antikörpern gegen tumorspezifische Oberflächenproteine inkubiert. Die markierten Zellen werden zweifach mit PBS gewaschen und im Anschluß werden 10⁷ Zellen in 1 ml PBS resuspendiert. Für die Isolierung der Tumorzellen wird ein FACS Vantage SE-Durchflußzytometer (Becton Dickinson) verwendet. Über das CellQuest Programm erfolgen Datenaufnahme, Instrumentenkontrolle und Datenauswertung. Die sortierten Zellen werden in ein 1,5 ml-Reaktionsgefäß (gefüllt mit 1 ml PBS) überführt. Die RNS kann dann wie oben beschrieben isoliert werden.
   Alternativ kann auch die isolierte Fraktion der mononukleären Blutkörperchen, die nach einem der obigen Verfahren isoliert wurden, in Trizol-Reagenz (Gibco BRL, NY, USA) lysiert und mittels Pipette homogenisiert werden. Nach Chloroformextraktion wird die RNA-haltige wäßrige Phase in Isopropanol bei -80°C gefällt. Nach zweimaligem Waschen in 80%igem Ethanol wird das Pellet an der Luft getrocknet und anschließend in RNase-freiem Wasser resuspendiert.
   An diese Isolation der RNS schließt sich dann die reverse Transkription sowie der mRNA-Nachweis wie oben beschrieben an.
d) Anreicherung von Tumorzellen durch immunomagnetische Separation.

Die Expression spezieller Oberflächenproteine unterscheidet Tumorzellen von nichttransformierten Zellen dieses Zelltyps. Da sich dieses spezielle Muster der Oberflächen-Antigene bei Tumorzellen auch von den Blutzell-typischen Mustern unterscheidet, können Tumorzellen im Blut unterschieden werden. Um Tumorzellen zu identifizieren, werden Antikörper, die diese speziellen Oberflächenproteine spezifisch erkennen, als Werkzeuge genutzt. Die spezifische Antikörperbindung wird für das erfindungsgemäße Verfahren nutzbar gemacht. An pseudomagnetische Partikel, die eine definierte Anzahl an chemisch aktivierten Stellen auf ihrer Oberfläche besitzen, werden Antikörper kovalent gekoppelt. Über die Spezifität der Antikörper wird die Spezifität der Trennung bestimmt. Eine Tumorzellen enthaltende Blutprobe wird mit Antikörper gekoppelte Magnetpartikel versetzt; als Antikörper werden in verschiedenen Beispielen zwei verschiedene Mischungen von Antikörpern verwendet; dann werden Partikel und Blut relativ zueinander bewegt, beispielsweise durch "Über-Kopf-Rotierer" in einem geschlossenen Behälter befindlicher Proben oder durch Bewegung der Partikel aufgrund von wechselnden Magnetfeldern. Jene (Tumor-)Zellen, die von den Festphase-gebundenen Antikörpern erkannt und damit fest gebunden werden, folgen der Bewegung der Partikel. Hierdurch ist es möglich, bei Anlegen eines magnetischen Feldes, die Partikel mit den daran gebundenen Zellen aus dem Blut herauszuziehen(z.B. an die Wand des Trenngefäßes). Das auf diese Weise Tumorzellen-depletierte Blut kann gegen andere Lösungen ausgetauscht werden, wobei die über Magnetpartikel separierten Zellen bis zum Abschalten/Entfernen des Magnetfeldes vor Ort verbleiben und für weitere Anwendungen zur Verfügung stehen.

**Tabelle 9: Antikörper-Mischung 1**

| **Antigen** | **Klon** | **Konzentration** |
|---|---|---|
| Epith.Rel.Antigen | MOC-31 (Fa. Novocastra) | 1,25 µl/10⁶ Zellen |
| Epitheliales Antigen | Ber-EP 4 (Fa. DAKO) | 0,924 µg/10⁶ Zellen |

Mittels der Antikörpermischung in Tabelle 9 werden ganz allgemein Tumorzellen jedoch mit hoher Spezifität erfaßt. Dies beruht auf der selektiven Expression bestimmter Oberflächenproteine, die Krebszellen von anderen Zellen unterscheidet.

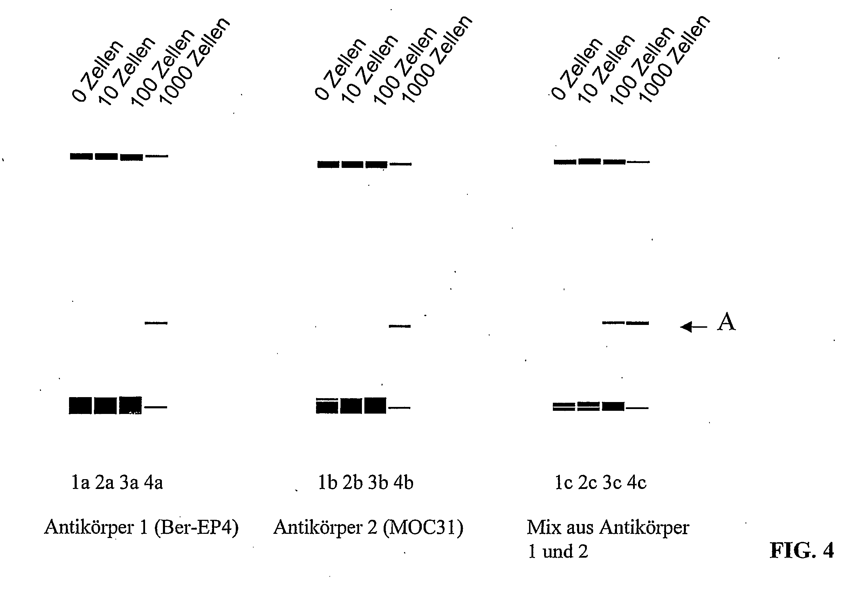

Durch den Einsatz der Antikörpermischung wird ganz grundsätzlich im Vergleich zu separat eingesetzten Antikörpern bei der Zelltrennung unabhängig von der angewendeten Methode eine erhöhte Sensitivität nachgewiesen. Dies zeigt sich in Fig. 4, bei der im Teilbild A mit dem Antikörper BER-EP4 belegte magnetische Partikel, in dem Teilbild B mit dem Antikörper MOC-31 belegte magnetische Partikel und in dem Teilbild C ein Mix aus Partikeln die jeweils separat mit einem Antikörper belegt wird, eingesetzt wurden.

Für jeden der Antikörper bzw. der Antikörpermischungen wurden insgesamt vier Messungen durchgeführt, bei denen 0, 10, 100 bzw. 1000 Karzinom-Zellen in 10 ml Blut inokuliert wurden. Die Spuren 1a bis 4a, 1b bis 4b bzw. 1c bis 4c zeigen dann den Nachweis von RNA nach RNA-Präparation und RT-PCR mit Tumormarkerspezifischen Primern wie oben beschrieben für Proben von jeweils 1 µl Volumen. Die Fig. 4 wurde dabei mittels elektrophoretischer Trennung in einem Agilent^{™} Bioanalyser 2100 gemäß Herstellerangaben ermittelt.

Bei Verwendung von lediglich mit einem Antikörper markierten magnetischen Partikeln wie in den Fign. 4A und 4B wurde lediglich bei einem Gehalt von 1000 Zellen ein Nachweis positiv möglich. Bei Verwendung einer Antikörpermischung wie in Fig. 4C wurde bereits ein Nachweis bei nur 100 Zellen und damit um den Faktor 10 sensitiver erbracht.

Bei diesem Beispiel wurden experimentelle Ergebnisse gezeigt, die nicht die maximal mögliche Sensitivität darstellen, sondern beispielhaft die mit dem erfindungsgemäßen Verfahren erreichbare Sensitivitätserhöhung demonstrieren sollen.

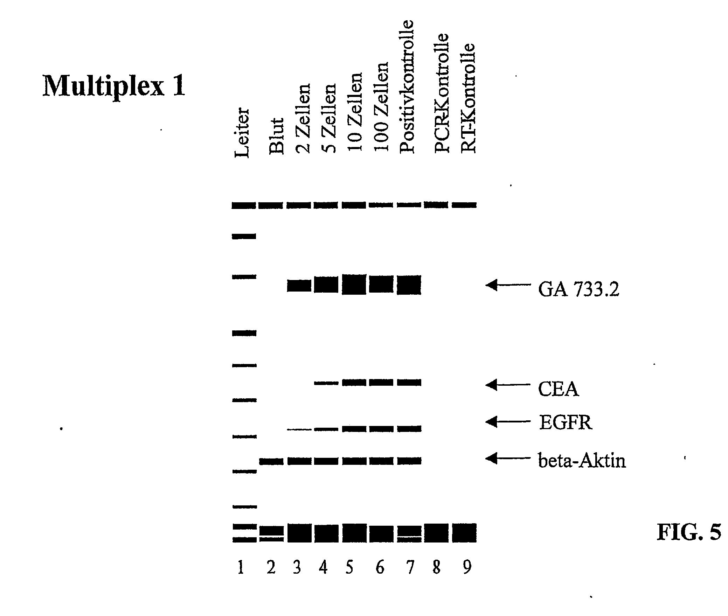

Die Fign. 5 bis 7 zeigen den Nachweis von Markerkombinationen, wobei in den folgenden Tabellen für die Fign. 5 bis 7 die jeweiligen Bedingungen für die drei Multiplexreaktionen angegeben-sind.

### Multiplex 1 (Fig. 5)

| | | | | |
|---|---|---|---|---|
| **Marker** | **EGFR** | **CEA** | **GA733.2** | **Aktin** |
| **Konz. Primer** | **750 nM** | **750 nM** | **500 nM** | **200 nM** |

Temperatur: 58 °C
40 Zyklen

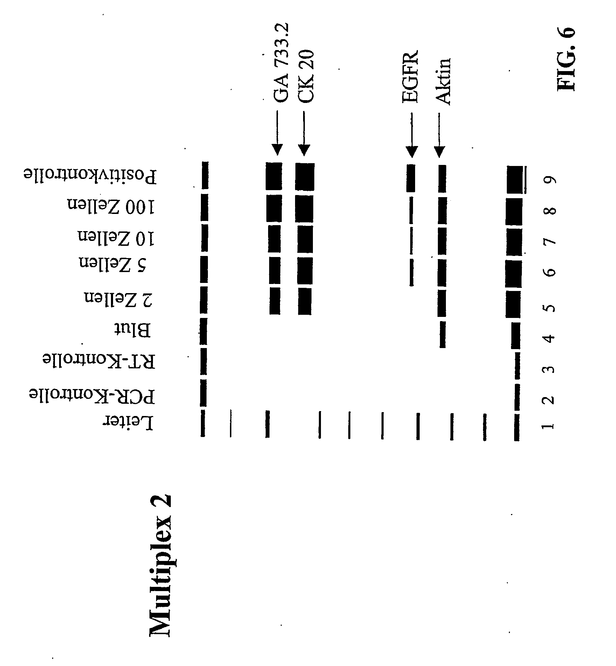

### Multiplex 2 (Fig. 6)

| | | | | |
|---|---|---|---|---|
| **Marker** | **EGFR** | **CK20** | **GA733.2** | **Aktin** |
| **Konz. Primer** | **1 µM** | **500 nM** | **250 nM** | **200 nM** |

Temperatur: 58 °C
35 Zyklen

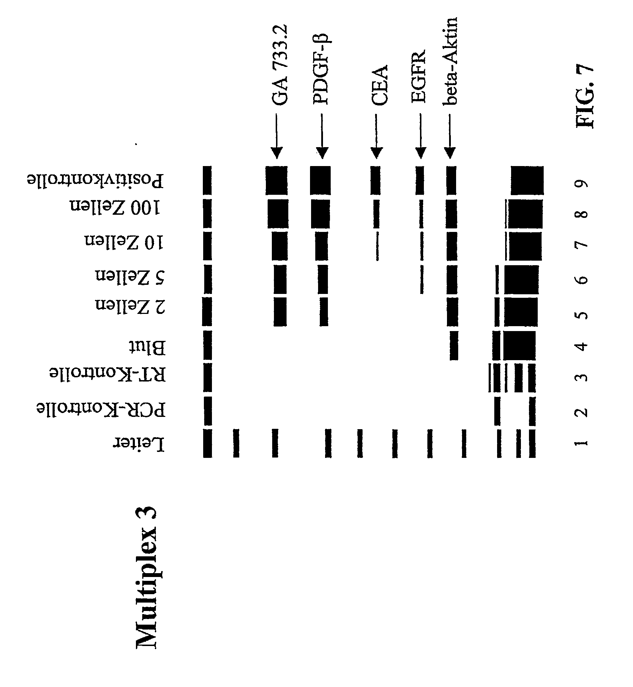

### Multiplex 3 (Fig. 7)

| | | | | | |
|---|---|---|---|---|---|
| **Marker** | **EGFR** | **CEA** | **PDGF-β** | **GA733.2** | **Aktin** |
| **Konz. Primer** | **1 µM** | **1 µM** | **500 nM** | **250 nM** | **200 nM** |

Temperatur: 58 °C
35 Zyklen

Fig. 5 zeigt den Nachweis von Darmkrebszellen, die in Blut inokuliert wurden. Dabei erfolgte die Selektion der Zellen mit den zwei Antikörpern BER-EP4 und MOC-31. Der molekularbiologische Nachweisschritt erfolgte mit den mRNS-Markern GA733.2 (Human carcinomaassociated antigen GA733-2 gen), CEA (Human carcinoembrionic antigen CEA gen) und EGF-R (Human epidermal growth factor receptor gen). Wie zu erkennen ist, waren bis herab zu zwei Zellen in 5 ml Blut zu detektieren.

Fig. 6 zeigt eine ähnliche Bestimmung mit den Tumormarkern GA733.2, CK20 (Homo sapiens gen for cytoceratin 20) sowie EGF-R. Wie zu erkennen ist, wurden auch hier bis herab zu zwei inokulierten Darmtumorzellen in 5 ml Blut sicher detektiert.

Fig. 7 zeigt den Nachweis mittels der parallelen Bestimmung der mRNA der Marker GA733.2, PDGF-β (Platelet derived growth faktor), CEA und EGF-R. Auch hier wurden wiederum bis herab zu 2 inokulierten Zellen pro 5 ml Blut sicher erfaßt.

Das erfindungsgemäße Diagnose-Kit und das erfindungsgemäße Verfahren ermöglicht es weiterhin, die sortierten und separierten Zellen anschließend beliebig weiter zu verwenden. Beispielsweise können diese in ein geeignetes Zellkulturmedium eingesetzt und in situ kultiviert werden.

Da die Zellen nach der Trennung intakt sind, bleiben auch die Eigenschaften der Zellmembran und des Zellkerns erhalten. Dies eröffnet die Möglichkeit, mikroskopisch die Expression weiterer Oberflächenmarker untersuchen oder auch Chromosomen-Analysen durchzuführen. Die sortierten Zellen werden dazu auf Objektträger aufgebracht. Der Nachweis weiterer Oberflächenmarker kann cytochemisch oder über Fluoreszenzmikroskopie erfolgen. Ebenso können genetische Analysen durchgeführt werden, wie beispielsweise Chromosomen-analysen mittels FISH (Flurorescence in situ hybridisation) oder Karyogramm-Erstellung.

### (Annex)

## Patentansprüche

1. Verfahren zur Diagnostik oder Behandlungskontrolle von Darmkrebs bei einem Menschen, **dadurch gekennzeichnet, dass** in einer Blutprobe des Menschen das Vorhandensein oder Fehlen von mindestens drei verschiedenen mRNS erfasst wird, die für die Tumormarkerproteine EGF-R, CEA und GA733.2 kodieren und bei Vorhandensein zumindest einer der mRNS auf das Vorhandensein von Darmtumorzellen in der Blutprobe und damit auf mögliche Metastasierung geschlossen wird.

2. Verfahren nach einem der vorhergehenden Ansprüche, **dadurch gekennzeichnet, dass** aus der Blutprobe Tumorzellen abgetrennt bzw. angereichert werden und der Nachweis an diesen Tumorzellen erfolgt.

3. Verfahren nach dem vorhergehenden Anspruch, **dadurch gekennzeichnet, dass** die Darmtumorzellen mittels für Tumorzellen allgemein spezifischer Antikörper und/oder mittels für Darmtumorzellen spezifischer Antikörper oder Mischungen derartiger Antikörper abgetrennt bzw. angereichert werden.

4. Verfahren nach einem der vorhergehenden Ansprüche, **dadurch gekennzeichnet, dass** die zur Abtrennung der Kolontumorzellen verwendeten Antikörper oder Antikörperderivate Bindungsstellen aufweisen, die an Epitope eines epithelialen Antigens und/oder eines epithelialen Membranantigens binden.

5. Verfahren nach dem vorhergehenden Anspruch, **dadurch gekennzeichnet, dass** als Antikörper MOC-31 und/oder Ber-EP4 verwendet werden.

6. Verfahren nach einem der beiden vorhergehenden Ansprüche, **dadurch gekennzeichnet, dass** die Darmtumorzellen mittels an Magnetpartikel gebundener Antikörper abgetrennt bzw. angereichert werden.

7. Verfahren nach Anspruch 3, **dadurch gekennzeichnet, dass** die Darmtumorzellen mittels fluoreszenzassoziierter Durchflusszytometrie, Dichtegradientenzentrifugation und/oder Zentrifugation nach Erythrozytenlyse abgetrennt bzw. angereichert werden.

8. Verfahren nach Anspruch 7, **dadurch gekennzeichnet, dass** eine Zentrifugation der Blutprobe zur Pelletierung der in ihr enthaltenen Leukozyten durchgeführt wird.

9. Verfahren nach Anspruch 7, **dadurch gekennzeichnet, dass** die RNS-haltigen Bestandteile der Probe mittels Lyse der darin enthaltenen Erythrozyten und anschließender Pelletierung der nichtlysierten Leukozyten aufkonzentriert werden.

10. Verfahren nach Anspruch 7, **dadurch gekennzeichnet, dass** die RNS-haltigen Bestandteile durch mindestens eine Dichtegradienten-Zentrifugation der Blutprobe zur Abseparierung und Gewinnung der in ihr enthaltenen mononukleären Blutzellen aufkonzentriert werden.

11. Verfahren nach einem der Ansprüche 7 bis 10, **dadurch gekennzeichnet, dass** die gewonnenen mononukleären Blutzellen mit fluoreszenzmarkierten Antikörpern markiert und mittels fluoreszenzaktivierter Zellsortierung (FACS) von der Probe abgetrennt und gewonnen werden.

12. Verfahren nach einem der Ansprüche 7 bis 11, **dadurch gekennzeichnet, dass** die mononukleären Zellen der gewonnenen Fraktion lysiert und die mRNS abgetrennt wird.

13. Verfahren nach Anspruch 1, **dadurch gekennzeichnet, dass** die RNS (Gesamt-RNS oder mRNS) in herkömmlicher Weise unmittelbar aus der Vollblutprobe isoliert wird.

14. Verfahren nach dem vorhergehenden Anspruch, **dadurch gekennzeichnet, dass** anschließend an die Isolation der mRNS ein DNS-Verdau durchgeführt wird.

15. Verfahren nach einem der vorhergehenden Ansprüche, **dadurch gekennzeichnet, dass** die gewonnene mRNS in cDNS revers transkribiert wird und anschließend das Vorhandensein oder Fehlen der cDNS erfasst wird, die dem Tumormarkerprotein zugeordnet ist.

16. Verfahren nach dem vorhergehenden Anspruch, **dadurch gekennzeichnet, dass** zumindest ein vorbestimmter Abschnitt der cDNS durch PolymeraseKettenreaktion ("PCR") vervielfältigt wird.

17. Verfahren nach dem vorhergehenden Anspruch, **dadurch gekennzeichnet, dass** zur Vervielfältigung der cDNS ein oder mehrere Oligonukleotid-Paare verwendet werden, die die folgenden Sequenzen aufweisen:
AACCCATGAGGCGGAGCAGAATGA und CGTTGGCGATGCATTTTAAGCTCT
und/oder
AGTCGGGCTCTGGAGGAAAAGAAA und GATCATAATTCCTCTGCACATAGG
und/oder
ATCTCCAAGGCCTGAATAAGGTCT und
CCTCAGTTCCTTTTAATTCTTCAGT
und/oder
AGAAATGACGCAAGAGCCTATGTA und
AACTTGTGTGTGTTGCTGCGGTAT
und/oder
AATCGTCAATGCCAGTGTACTTCA und
TAACGCGTTGTGATCTCCTTCTGA
und/oder
TCTCTCTGCTGCTACCTGCGTCTG und
GTTGGCGTTGGTGCGGTCTATGAG.

18. Verfahren nach einem der vorhergehenden Ansprüche, **dadurch gekennzeichnet, dass** als interne Kontrolle die mRNS des Proteins β-Aktin bestimmt wird.

19. Verfahren nach dem vorhergehenden Anspruch, **dadurch gekennzeichnet, dass** die mRNS für β-Aktin in cDNS revers transkribiert und ein Abschnitt der cDNS mittels Polymerasekettenreaktion vervielfältigt wird.

20. Verfahren nach dem vorhergehenden Anspruch, **dadurch gekennzeichnet, dass** zur Vervielfältigung der cDNS des β-Aktin ein Oligonukleotid-Paar verwendet wird, wobei die Oligonukleotide des Paares die folgenden Sequenzen aufweisen:
CTGGAGAAGAGCTACGAGCTGCCT und
ACAGGACTCCATGCCCAGGAAGGA.

21. Verfahren nach einem der Ansprüche 16 bis 20, **dadurch gekennzeichnet, dass** der vervielfältigte cDNS-Abschnitt durch geeignete Restriktionsenzyme verdaut und anhand der erzeugten cDNS-Bruchstücke das Vorhandensein oder Fehlen der mRNS eines Tumormarkerproteins bestimmt wird.

22. Verfahren nach einem der Ansprüche 16 bis 20, **dadurch gekennzeichnet, dass** zum Nachweis der amplifizierten cDNS-Abschnitte eine Gelelektrophorese der PCR-Produkte durchgeführt wird.

23. Verfahren nach einem der Ansprüche 16 bis 20, **dadurch gekennzeichnet, dass** zum Nachweis der amplifizierten cDNS-Abschnitte eine Fragmentanalyse durchgeführt wird.

24. Verfahren nach einem der Ansprüche 16 bis 20, **dadurch gekennzeichnet, dass** während des Verlaufs der Polymerasekettenreaktion die von den Produkten erzeugte Fluoreszenz erfasst und die Produktentwicklung erfasst wird (fluoreszenzbasierte Echtzeit-PCR).

25. Verfahren nach einem der Ansprüche 16 bis 20, **dadurch gekennzeichnet, dass** zum Nachweis der mRNS oder cDNS ein Nukleotid-Mikroarray verwendet wird, wobei das Nukleotid-Mikroarray eine Anordnung von mehreren, voneinander getrennten Zellen aufweist, wobei in mindestens drei Zellen jeweils verschiedene Oligonukleotide angeordnet sind, die mit jeweils verschiedenen DNS-Abschnitten hybridisieren, die Teil der cDNS zu je einem der Tumormarkerproteine EGF-R, CEA und GA733.2 sind.

26. Verfahren nach dem vorhergehenden Anspruch, **dadurch gekennzeichnet, dass** zum Nachweis der amplifizierten cDNS das PCR-Produkt auf das Nukleotid-Mikroarray aufgetragen wird.

27. Diagnose-Kit zur Diagnostik oder Behandlungskontrolle von Darmkrebs mit mindestens drei Paaren Oligonukleotide (Reverseprimer, Forwardprimer), wobei die beiden Oligonukleotide jedes Paares als Primer zur Amplifikation mittels Polymerasekettenreaktion jeweils eines der beiden komplementären Stränge eines gesuchten DNS-Abschnittes geeignet sind, und wobei der gesuchte DNS-Abschnitt für jedes der Primerpaare Teil der cDNS zu je einem der Tumormarkerproteine EGF-R, CEA und GA733.2 ist.

28. Diagnose-Kit nach dem vorhergehenden Anspruch, **dadurch gekennzeichnet, dass** es ein weiteres Paar Oligonukleotide enthält, die jeweils als Primer zur Amplifikation zumindest eines Abschnitts eines der beiden komplementären Stränge der cDNS zu dem Protein β-Aktin als interne Kontrolle geeignet sind.

29. Diagnose-Kit nach einem der Ansprüche 27 bis 28, **dadurch gekennzeichnet, dass** die beiden Oligonukleotide eines Paares paarweise jeweils eines der folgenden Sequenzpaare aufweisen:
AACCCATGAGGCGGAGCAGAATGA und CGTTGGCGATGCATTTTAAGCTCT
und/oder
AGTCGGGCTCTGGAGGAAAAGAAA und GATCATAATTCCTCTGCACATAGG
und/oder
ATCTCCAAGGCCTGAATAAGGTCT und
CCTCAGTTCCTTTTAATTCTTCAGT
und/oder
AGAAATGACGCAAGAGCCTATGTA und
AACTTGTGTGTGTTGCTGCGGTAT
und/oder
AATCGTCAATGCCAGTGTACTTCA und
TAACGCGTTGTGATCTCCTTCTGA
und/oder
TCTCTCTGCTGOTACCTGCGTCTG und
GTTGGCGTTGGTGCGGTCTATGAG.

30. Diagnose-Kit nach einem der Ansprüche 27 bis 29, **dadurch gekennzeichnet, dass** zumindest jeweils eines der beiden Oligonukleotide eines Paares von Oligonukleotiden mit Fluorophoren markiert ist.

31. Diagnose-Kit nach dem vorhergehenden Anspruch, **dadurch gekennzeichnet, dass** die Oligonukleotide verschiedener Paare mit verschiedenen Fluorophoren markiert sind.

32. Diagnose-Kit nach einem der Ansprüche 27 bis 31, **dadurch gekennzeichnet, dass** es zur Amplifikation der cDNS zu β-Aktin ein Paar Oligonukleotide mit den folgenden Sequenzen enthält:
CTGGAGAAGAGCTACGAGCTGCCT und
ACAGGACTCCATGCCCAGGAAGGA.

33. Diagnose-Kit nach dem vorhergehenden Anspruch, **dadurch gekennzeichnet, dass** zumindest jeweils eines der beiden Oligonukleotide des Paares zur Amplifikation der cDNS zu β-Aktin mit Fluorophoren markiert ist.

34. Diagnose-Kit nach einem der Ansprüche 27 bis 33, **dadurch gekennzeichnet, dass** es die zur Durchführung einer Polymerasekettenreaktion erforderlichen Substanzen enthält.

35. Diagnose-Kit nach einem der Ansprüche 27 bis 34, **dadurch gekennzeichnet, dass** es als zur Durchführung einer Polymerasekettenreaktion erforderliche Substanzen eine Pufferlösung, Magnesiumchlorid, Desoxynukleotid-Triphosphate sowie eine hitzestabile Polymerase enthält.

36. Diagnose-Kit nach dem vorhergehenden Anspruch, **dadurch gekennzeichnet, dass** es als hitzestabile Polymerase eine Polymerase aus Thermus aquaticus (Taq-Polymerase) enthält.

37. Diagnose-Kit nach einem der Ansprüche 27 bis 36, **dadurch gekennzeichnet, dass** es als Positivkontrolle eine DNS-Probe mit dem jeweils gesuchten DNS-Abschnitt enthält.

38. Diagnose-Kit nach einem der Ansprüche 27 bis 37, **dadurch gekennzeichnet, dass** es eine Anleitung zur Durchführung der Polymerasekettenreaktion und/oder eine Anleitung zur Durchführung einer Fragmentanalyse enthält.

39. Diagnose-Kit nach einem der Ansprüche 27 bis 38, **dadurch gekennzeichnet, dass** es ein Schema zur Auswertung der erhaltenen Messergebnisse enthält.

40. Diagnose-Kit nach einem der Ansprüche 27 bis 39, **dadurch gekennzeichnet, dass** es einen Mikroarray (DNS-Chip) enthält, wobei der Array eine Anzahl voneinander getrennter Zellen (Felder) aufweist und in mindestens drei Zellen des Mikroarrays jeweils ein Oligonukleotid angeordnet ist, das mit einem der gesuchten DNS-Abschnitte hybridisiert.

41. Diagnose-Kit nach dem vorhergehenden Anspruch, **dadurch gekennzeichnet, dass** in mindestens einer weiteren Zelle des Mikroarrays ein weiteres Oligonukleotid angeordnet ist und die Sequenz des Oligonukleotids, das in der mindestens einen Zelle angeordnet ist, sich von der Sequenz des weiteren Oligonukleotids unterscheidet.

42. Verwendung eines Diagnose-Kits und/oder eines Verfahrens nach einem der vorhergehenden Ansprüche zur Diagnose von Erkrankungen oder Metastasierungen oder zur Behandlungskontrolle bei Darmkrebs.

## Claims

1. Method for diagnosis or treatment control of intestinal carcinoma in humans, **characterised in that** the presence or absence of at least three different mRNS in a human blood sample are recorded, which code for the tumour marker proteins EGF-R, CEA, and GA733.2, and if at least one of the mRNS is present, deduction that intestinal tumour cells are present in the blood sample, and therefore possible metastases.

2. Method according to the preceding Claim, **characterised in that** tumour cells are separated out of the blood sample, e.g. enriched, and the diagnosis is carried out on these tumour cells.

3. Method according to the preceding Claim, **characterised in that** the intestinal tumour cells are separated out, e.g. enriched by means of antibodies generally specific for tumour cells and/or by means of antibodies or mixtures of said antibodies specific for intestinal tumour cells.

4. Method according to one of the preceding Claims, **characterised in that** the antibodies or antibody derivatives used for separating out the colonic tumour cells comprise connection points which connect to epitopes of an epithelial antigen and/or an epithelial membrane antigen.

5. Method according to the preceding Claim, **characterised in that** MOC-31 and/or Ber-EP4 are used as antibodies.

6. Method according to one of the two preceding Claims, **characterised in that** the intestinal tumour cells are separated out, e.g. enriched by means of antibodies connected to magnetic particles.

7. Method according to Claim 3, **characterised in that** the intestinal tumour cells are separated out, e.g. enriched by means of fluorescence associated flow zytometry, density gradient centrifugation and/or centrifugation following erythrozytenlysis.

8. Method according to Claim 7, **characterised in that** a centrifugation of the blood sample is carried out for pelleting the leukocytes contained within the same.

9. Method according to Claim 7, **characterised in that** the RNS containing components of the sample are concentrated by means of lyses of the erythrocytes contained within the same and subsequent pelleting of the non-lysised leukocytes.

10. Method according to Claim 7, **characterised in that** the RNS containing components are concentrated by means of at least one density gradient centrifugation of the blood sample for separating out and harvesting the mononuclear blood cells contained within the same.

11. Method according to one of the Claims 7 to 10, **characterised in that** the harvested mononuclear blood cells are marked with fluorescence marked antibodies and separated out and harvested from the sample by means of fluorescence activated cell sorting (FACS).

12. Method according to one of the Claims 7 to 11, **characterised in that** the mononuclear cells of the harvested fraction are lysised and the mRNS separated out.

13. Method according to Claim 1, **characterised in that** the RNS (total RNS or mRNS) is isolated in the conventional way directly from the whole blood sample.

14. Method according to one of the preceding Claims, **characterised in that** a DNS digestion is carried out following the isolation of the mRNS.

15. Method according to one of the preceding Claims, **characterised in that** the harvested mRNS is reverse transcribed in cDNS and the presence or absence of the cDNS subsequently recorded, which is associated with the tumour marker protein.

16. Method according to the preceding Claim, **characterised in that** at least one pre-determined section of the cDNS is multiplied by means of polymerase chain reaction ("PCR").

17. Method according to the preceding Claim, **characterised in that** one or more oligonucleotide pairs with the following sequences are used for multiplying the cDNS:
AACCCATGAGGCGGAGCAGAATGA and CGTTGGCGATGCATTTTAAGCTCT
and/or
AGTCGGGCTCTGGAGGAAAAGAAA and GATCATAATTCCTCTGA-CATAGG
and/or
ATCTCCAAGGCCTGAATAAGGTCT and
CCTCAGTTCCTTTTAATTCTTCAGT
and/or
AGAAATGACGCAAGAGCCTATGTA and
AACTTGTGTGTGTTGCTGCGGTAT and/or
AATCGTCAATGCCAGTGTACTTCA and TAACGCGTTGTGATCTCCTTCTGA
and/or
TCTCTCTGCTGCTACCTGCGTCTG and
GTTGGCGTTGGTGCGGTCTATGAG.

18. Method according to one of the preceding Claims, **characterised in that** the mRNS of the protein β-actin is determined as an internal control.

19. Method according to the preceding Claim, **characterised in that** the mRNS for β-actin is reverse transcribed in cDNS and a section of the cDNS multiplied by means of polymerase chain reaction.

20. Method according to the preceding Claim, **characterised in that** an oligonucleotide pair is used for multiplying the cDNS of the β-actin, whereby the oligonucleotides of the pair comprise the following sequences:
CTGGAGAAGAGCTACGAGCTGCCT and
ACAGGACTCCATGCCCAGGAAGGA.

21. Method according to one of the Claims 16 to 20, **characterised in that** the multiplied cDNS section is digested by means of suitable restriction enzymes and the presence or absence of the mRNS of a tumour marker protein determined by means of the harvested cDNS fractions.

22. Method according to one of the Claims 16 to 20, **characterised in that** a gel electrophoresis of the PCR products is carried out for detecting the amplified cDNS sections.

23. Method according to one of the Claims 16 to 20, **characterised in that** a fragment analysis is carried out for detecting the amplified cDNS sections.

24. Method according to one of the Claims 16 to 20, **characterised in that** the fluorescence generated by the products during the duration of the polymerase chain reaction is recorded, and the product development recorded (fluorescence based real time PCR).

25. Method according to one of the Claims 16 to 20, **characterised in that** a nucleotide micro array is used for detecting the mRNS or cDNS, whereby the nucleotide micro array comprises an arrangement of several cells separated from each other, whereby different oligonucleotides are arranged in at least three cells each, which each hybridise with different DNS sections, which form a part of the cDNS for each of the tumour marker proteins EGF-R, CEA, and GA733.2.

26. Method according to one of the preceding Claims, **characterised in that** for detecting the amplified cDNS the PCR product is applied to the nucleotide micro array.

27. Diagnosis kit for the diagnosis or treatment control of intestine carcinoma with at least three pairs of oligonucleotides (reverse primer, forward primer), whereby the two oligonucleotides of each pair are suited as primer for amplification by means of polymerase chain reaction of one of the two complementary strands of a desired DNS section each, and whereby the desired DNS section for each of the primer pairs is a part of the cDNS for each of the tumour marker proteins EGF-R, CEA, and GA733.2.

28. Diagnosis kit according to the preceding Claim, **characterised in that** the same comprises a further pair of oligonucleotides, which is suited as a primer for amplification of at least one section of one of the two complementary strands of the cDNS for the protein β-actin as an internal control.

29. Diagnosis kit according to one of the Claims 27 or 28, **characterised in that** the two oligonucleotides of one pair each comprise one of the following sequence pairs as a pair:
AACCCATGAGGCGGAGCAGAATGA and CGTTGGCGATGCATTTTAAGCTCT
and/or
AGTCGGGCTCTGGAGGAAAAGAAA and GATCATAATTCCTCTGCACATAGG
and/or
ATCTCCAAGGCCTGAATAAGGTCT and
CCTCAGTTCCTTTTAATTCTTCAGT
and/or
AGAAATGACGCAAGAGCCTATGTA and
AACTTGTGTGTGTTGCTGCGGTAT
and/or
AATCGTCAATGCCAGTGTACTTCA and
TAACGCGTTGTGATCTCCTTCTGA
and/or
TCTCTCTGCTGOTACCTGCGTCTG and
GTTGGCGTTGGTGCGGTCTATGAG.

30. Diagnosis kit according to one of the claims 27 to 29, **characterised in that** at least one each of the two oligonucleotides of a pair of oligonucleotides is marked with fluorophores.

31. Diagnosis kit according to the preceding Claim, **characterised in that** the oligonucleotides of different pairs are marked with different fluorophores.

32. Diagnosis kit according to one of the Claims 27 to 31, **characterised in that** the same comprises a pair of oligonucleotides with the following sequences for the amplification of the cDNS for β-actin:
CTGGAGAAGAGCTACGAGCTGCCT and
ACAGGACTCCATGCCCAGGAAGGA.

33. Diagnosis kit according to the preceding Claim, **characterised in that** at least one of the two oligonucleotides of the pair for amplification of the cDNS for β-actin each is marked with fluorophore.

34. Diagnosis kit according to one of the Claims 27 to 33, **characterised in that** the same comprises the substances required for carrying out a polymerase chain reaction.

35. Diagnosis kit according to one of the Claims 27 to 34, **characterised in that** the same comprises substances like a buffer solution, magnesium chloride, desoxynucleotide-triphosphate as well as a heat stable polymerase required for carrying out a polymerase chain reaction.

36. Diagnosis kit according to the preceding Claim, **characterised in that** the same comprises a polymerase of thermus aquaticus (taq-polymerase) as a heat stable polymerase.

37. Diagnosis kit according to one of the Claims 27 to 36, **characterised in that** the same comprises a DNS sample with the relevant desired DNS section as a positive control.

38. Diagnosis kit according to one of the Claims 27 to 37, **characterised in that** the same comprises instructions for carrying out the polymerase chain reaction and/or instructions for carrying out a fragment analysis.

39. Diagnosis kit according to one of the Claims 27 to 38, **characterised in that** the same comprises a plan for evaluating the recorded measurement results.

40. Diagnosis kit according to one of the Claims 27 to 39, **characterised in that** the same comprises a micro array (DNS chip), whereby the array comprises a number of cells (fields) separate from each other, and **in that** one oligonucleotide is located in at least three cells of the micro array, which hybridises with one of the desired DNS sections.

41. Diagnosis kit according to the preceding Claim, **characterised in that** a further oligonucleotide is located in at least one further cell of the micro array, and **in that** the sequence of the oligonucleotide located in at least one cell is different from the sequence of the further oligonucleotide.

42. Use of a diagnosis kit and/or a method according to one of the preceding Claims for the diagnosis of diseases or metastases, or for the treatment control of intestinal carcinoma.

## Revendications

1. Procédé pour le diagnostic ou pour le suivi du traitement du cancer de l'intestin chez l'homme, **caractérisé en ce qu'**on détecte dans un échantillon de sang humain la présence ou l'absence d'au moins trois ARNm codant pour les protéines-marqueurs tumorales EGF-R, CEA et GA733.2, et **en ce qu'**en cas de présence d'au moins un des ARNm, on conclut à la présence de cellules tumorales de l'intestin dans l'échantillon de sang, et donc au développement possible de métastases.

2. Procédé selon l'une des revendications précédentes, **caractérisé en ce que** des cellules tumorales provenant de l'échantillon de sang sont séparées et/ou enrichies, et **en ce que** la détection s'effectue sur ces cellules tumorales.

3. Procédé selon la revendication précédente, **caractérisé en ce que** les cellules tumorales de l'intestin sont séparées et/ou enrichies au moyen d'anticorps spécifiques pour les cellules tumorales en général, et/ou au moyen d'anticorps spécifiques pour les cellules tumorales de l'intestin, ou au moyen de mélanges de ces anticorps.

4. Procédé selon une des revendications précédentes, **caractérisé en ce que** les anticorps ou les dérivés d'anticorps utilisés pour la séparation des cellules tumorales du colon présentent des sites de liaison qui se lient à l'épitope d'un antigène épithélial et ou d'un antigène épithélial membranaire.

5. Procédé selon la revendication précédente, **caractérisé en ce qu'**on utilise comme anticorps les anticorps MOC-31 et/ou Ber-EP4.

6. Procédé selon l'une des deux revendications précédentes, **caractérisé en ce que** les cellules tumorales de l'intestin sont séparées et/ou enrichies au moyen d'un anticorps lié à des particules magnétiques.

7. Procédé selon la revendication 3, **caractérisé en ce que** les cellules tumorales de l'intestin sont séparées et/ou enrichies par cytométrie de flux associée à la fluorescence, par centrifugation sur gradient de densité et/ou par centrifugation après lyse des érythrocytes.

8. Procédé selon la revendication 7, **caractérisé en ce qu'**on effectue une centrifugation de l'échantillon de sang pour la pelletisation des leucocytes qu'il contient.

9. Procédé selon la revendication 7, **caractérisé en ce que** les constituants de l'échantillon contenant de l'ARN sont concentrés par lyse des érythrocytes qu'ils contiennent, suivie de la pelletisation des leucocytes non lysés.

10. Procédé selon la revendication 7, **caractérisé en ce que** les constituants de l'échantillon contenant de l'ARN sont concentrés par au moins une centrifugation sur gradient de densité de l'échantillon de sang, pour la séparation et la récupération des cellules sanguines mononucléaires qu'il contient.

11. Procédé selon l'une des revendications 7 à 10, **caractérisé en ce que** les cellules sanguines mononucléaires sont marquées par des anticorps marqués par fluorescence et sont séparées de l'échantillon et récupérées par tri cellulaire basé sur la fluorescence (FACS).

12. Procédé selon l'une des revendications 7 à 11, **caractérisé en ce que** les cellules mononucléaires de la fraction récupérée sont lysées et l'ARNm est séparé.

13. Procédé selon la revendication 1, **caractérisé en ce que** l'ARN (ARN total ou ARNm) est isolé directement de l'échantillon complet de sang de manière traditionnelle.

14. Procédé selon la revendication précédente, **caractérisé en ce qu'**une digestion de l'ADN est effectuée à la suite de l'isolement de l'ARNm.

15. Procédé selon une des revendications précédentes, **caractérisé en ce que** l'ARNm extrait est transcrit en sens inverse en ADNc, après quoi on détecte la présence ou l'absence de l'ADNc qui est associé à la protéine-marqueur tumorale.

16. Procédé selon la revendication précédente, **caractérisé en ce qu'**au moins un segment prédéterminé de l'ADNc est répliqué par réaction en chaîne par polymérase (PCR).

17. Procédé selon la revendication précédente, **caractérisé en ce qu'**on utilise pour la réplication de l'ADNc une ou plusieurs paires d'oligonucléotides qui présentent les séquences ci-dessous :
AACCCATGAGGCGGAGCAGAATGAet
CGTTGGCGATGCATTTTAAGCTCT
et/ou
AGTCGGGCTCTGGAGGAAAAGAAA et
GATCATAATTCCTCTGCACATAGG
et/ou
ATCTCCAAGGCCTGAATAAGGTCT et
CCTCAGTTCCTTTTAATTCTTCAGT
et/ou
AGAAATGACGCAAGAGCCTATGTA et
AACTTGTGTGTGTTGCTGCGGTAT
et/ou
AATCGTCAATGCCAGTGTACTTCA et
TAACGCGTTGTGATCTCCTTCTGA
et/ou
TCTCTCTGCTGCTACCTGCGTCTG et
GTTGGCGTTGGTGCGGTCTATGAG;

18. Procédé selon l'une des revendications précédentes, **caractérisé en ce qu'**on détermine comme contrôle interne l'ARNm de la protéine bêta-actine.

19. Procédé selon la revendication précédente, **caractérisé en ce que** l'ARNm pour la protéine bêta-actine est transcrit en sens inverse en ADNc, et **en ce qu'**un segment de l'ADNc est répliqué par réaction en chaîne par polymérase.

20. Procédé selon la revendication précédente, **caractérisé en ce qu'**on utilise pour la réplication de l'ADNc de la protéine bêta-actine une paire d'oligonucléotides, les oligonucléotides de la paire présentant les séquences ci-dessous :
CTGGAGAAGAGCTACGAGCTGCCT et
ACAGGACTCCATGCCCAGGAAGGA.

21. Procédé selon l'une des revendications 16 à 20, **caractérisé en ce que** le segment de l'ADNc répliqué est digéré par des enzymes de restriction appropriés, et **en ce qu'**on détermine la présence ou l'absence de l'ARNm d'une protéine-marqueur tumorale à l'aide des fragments d'ADNc produits.

22. Procédé selon l'une des revendications 16 à 20, **caractérisé en ce qu'**on effectue une électrophorèse sur gel du produit de la PCR pour détecter les segments d'ADNc amplifiés.

23. Procédé selon l'une des revendications 16 à 20, **caractérisé en ce qu'**on effectue une analyse de fragment pour détecter les segments d'ADNc amplifiés.

24. Procédé selon l'une des revendications 16 à 20, **caractérisé en ce que** pendant le déroulement de la réaction en chaîne par polymérase, on détecte la fluorescence générée par les produits, et on détecte le développement des produits (PCR en temps réel basée sur la fluorescence).

25. Procédé selon l'une des revendications 16 à 20, **caractérisé en ce qu'**on utilise pour déceler l'ARNm ou l'ADNc une micro-matrice de nucléotides, laquelle micro-matrice de nucléotides présente un arrangement de plusieurs cellules séparées les unes des autres, moyennant quoi dans au moins trois cellules sont disposés différents oligonucléotides qui s'hybrident chacun avec différents segments d'ADN qui font partie de l'ADNc pour chacune des protéines-marqueurs tumorales EGF-R, CEA et GA733.2.

26. Procédé selon la revendication précédente, **caractérisé en ce que** pour détecter l'ADNc amplifié, le produit de la PCR est transféré sur la micro-matrice de nucléotides.

27. Kit de diagnostic pour le diagnostic ou le suivi du traitement du cancer de l'intestin, comprenant au moins trois paires d'oligonucléotides (reverse primer, forward primer), les deux oligonucléotides de chaque paire convenant en tant qu'amorce pour l'amplification au moyen de la réaction en chaîne par polymérase d'un des deux brins complémentaires d'un segment d'ADN analysé, et le segment d'ADN analysé étant, pour chacune des paires d'amorces, partie de l'ADNc pour chacune des protéines-marqueurs tumorales EGF-R, CEA et GA733.2.

28. Kit de diagnostic selon la revendication précédente, **caractérisé en ce qu'**il contient une autre paire d'oligonucléotides qui conviennent chacun comme amorce pour l'amplification d'au moins un segment d'un des deux brins complémentaires de l'ADNc pour la protéine bêta-actine en tant que contrôle interne.

29. Kit de diagnostic selon l'une des revendications 27 à 28, **caractérisé en ce que** les deux oligonucléotides d'une paire présentent chacun par paire une des paires de séquences ci-dessous :
AACCCATGAGGCGGAGCAGAATGAet
CGTTGGCGATGCATTTTAAGCTCT
et/ou
AGTCGGGCTCTGGAGGAAAAGAAA et
GATCATAATTCCTCTGCACATAGG
et/ou
ATCTCCAAGGCCTGAATAAGGTCT et
CCTCAGTTCCTTTTAATTCTTCAGT
et/ou
AGAAATGACGCAAGAGCCTATGTA et
AACTTGTGTGTGTTGCTGCGGTAT
et/ou
AATCGTCAATGCCAGTGTACTTCA et
TAACGCGTTGTGATCTCCTTCTGA
et/ou
TCTCTCTGCTGCTACCTGCGTCTG et
GTTGGCGTTGGTGCGGTCTATGAG;

30. Kit de diagnostic selon l'une des revendications 27 à 29, **caractérisé en ce qu'**au moins un de chacun des deux oligonucléotides d'une paire d'oligonucléotides est marqué par des fluorophores.

31. Kit de diagnostic selon la revendication précédente, **caractérisé en ce que** les oligonucléotides des différentes paires sont marqués avec des fluorophores différents.

32. Kit de diagnostic selon l'une des revendications 27 à 31, **caractérisé en ce que** qu'il contient, pour l'amplification de l'ADNc pour la protéine bêta-actine, une paire d'oligonucléotides présentant les séquences suivantes :
CTGGAGAAGAGCTACGAGCTGCCT et
ACAGGACTCCATGCCCAGGAAGGA

33. Kit de diagnostic selon la revendication précédente, **caractérisé en ce qu'**au moins un de chacun des deux oligonucléotides de la paire est marqué avec des fluorophores pour l'amplification de l'ADNc pour la protéine bêta-actine.

34. Kit de diagnostic selon l'une des revendications 27 à 33, **caractérisé en ce qu'**il contient les substances nécessaires pour la mise en oeuvre d'une réaction en chaîne par polymérase.

35. Kit de diagnostic selon l'une des revendications 27 à 34, **caractérisé en ce** q'il contient en tant que substances nécessaires pour la mise en oeuvre d'une réaction en chaîne par polymérase une solution tampon, du chlorure de magnésium, un désoxynucléotide triphosphate ainsi qu'une polymérase stable à la chaleur.

36. Kit de diagnostic selon la revendication précédente, **caractérisé en ce qu'**il contient en tant que polymérase stable à la chaleur une polymérase provenant de Thermus Aquaticus (Taq polymérase).

37. Kit de diagnostic selon l'une des revendications 27 à 36, **caractérisé en ce qu'**il contient en tant que contrôle positif un échantillon d'ADN comportant chacun des segments d'ADN analysés.

38. Kit de diagnostic selon l'une des revendications 27 à 37 **caractérisé en ce qu'**il contient un mode d'emploi pour la mise en oeuvre de la réaction en chaîne par polymérase et/ou un mode d'emploi pour la mise en oeuvre d'une analyse de fragment.

39. Kit de diagnostic selon l'une des revendications 27 à 38, **caractérisé en ce qu'**il contient un schéma pour l'évaluation des résultats de mesure obtenus.

40. Kit de diagnostic selon l'une des revendications 27 à 39, **caractérisé en ce qu'**il contient une micro-matrice (puce à ADN, laquelle micro-matrice présente un nombre de cellules (champs) séparées les unes des autres, et dans laquelle, dans chacune d'au moins trois cellules de la puce à ADN, est disposé un oligonucléotide qui s'hybride avec un des segments d'ADN analysés.

41. Kit de diagnostic selon la revendication précédente, **caractérisé en ce qu'**un autre oligonucléotide est disposé dans au moins une autre cellule de la micro-matrice, et **en ce que** la séquence de l'oligonucléotide qui est disposé dans la au moins une autre cellule diffère de la séquence de l'autre oligonucléotide.

42. Utilisation d'un kit de diagnostic et/ou d'un procédé selon l'une des revendications précédentes pour le diagnostic de maladies et de développement de métastases, ou pour le suivi du traitement du cancer de l'intestin.


### SEQUENZPROTOKOLL

<110> Adnagen AG
   Krehan, Alf-Andreas
   Steffens, Pia
   Waschütza, Stefanie
   Albert, Winfried
   Stefan, Monica
<120> Verfahren und Kit zur Diagnostik oder
   Behandlungskontrolle von Darmkrebs
<130> PCT/EP02/09983
<140> PCT/EP02/09983
   <141> 2002-09-06
<150> DE 10143775.5
   <151> 2001-09-06
<150> PCT/EP02/05489
   <151> 2002-05-17
<160> 14
<170> PatentIn Ver. 2.1
<210> 1
   <211> 24
   <212> DNA
   <213> Künstliche Sequenz
<220>
   <223> Beschreibung der künstlichen Sequenz:
   Oligonukleotid
<400> 1
   aatcgtcaat gccagtgtac ttca 24
<210> 2
   <211> 24
   <212> DNA
   <213> Künstliche Sequenz
<220>
   <223> Beschreibung der künstlichen Sequenz:
   Oligonukleotid
<400> 2
   taacgcgttg tgatctcctt ctga 24
<210> 3
   <211> 24
   <212> DNA
   <213> Künstliche Sequenz
<220>
   <223> Beschreibung der künstlichen Sequenz:
   Oligonukleotid
<400> 3
   agtcgggctc tggaggaaaa gaaa 24
<210> 4
   <211> 24
   <212> DNA
   <213> Künstliche Sequenz
<220>
   <223> Beschreibung der künstlichen Sequenz:
   Oligonukleotid
<400> 4
   gatcataatt cctctgcaca tagg 24
<210> 5
   <211> 24
   <212> DNA
   <213> Künstliche Sequenz
<220>
   <223> Beschreibung der künstlichen Sequenz:
   Oligonukleotid
<400> 5
   agaaatgacg caagagccta tgta 24
<210> 6
   <211> 24
   <212> DNA
   <213> Künstliche Sequenz
<220>
   <223> Beschreibung der künstlichen Sequenz:
   Oligonukleotid
<400> 6
   aacttgtgtg tgttgctgcg gtat 24
<210> 7
   <211> 24
   <212> DNA
   <213> Künstliche Sequenz
<220>
   <223> Beschreibung der künstlichen Sequenz:
   Oligonukleotid
<400> 7
   atctccaagg cctgaataag gtct 24
<210> 8
   <211> 25
   <212> DNA
   <213> Künstliche Sequenz
<220>
   <223> Beschreibung der künstlichen Sequenz:
   Oligonukleotid
<400> 8
   cctcagttcc ttttaattct tcagt 25
<210> 9
   <211> 24
   <212> DNA
   <213> Künstliche Sequenz
<220>
   <223> Beschreibung der künstlichen Sequenz:
   Oligonukleotid
<400> 9
   aacccatgag gcggagcaga atga 24
<210> 10
   <211> 24
   <212> DNA
   <213> Künstliche Sequenz
<220>
   <223> Beschreibung der künstlichen Sequenz:
   Oligonukleotid
<400> 10
   cgttggcgat gcattttaag ctct 24
<210> 11
   <211> 24
   <212> DNA
   <213> Künstliche Sequenz
<220>
   <223> Beschreibung der künstlichen Sequenz:
   Oligonukleotid
<400> 11
   tctctctgct gctacctgcg tctg 24
<210> 12
   <211> 24
   <212> DNA
   <213> Künstliche Sequenz
<220>
   <223> Beschreibung der künstlichen Sequenz:
   Oligonukleotid
<400> 12
   gttggcgttg gtgcggtcta tgag 24
<210> 13
   <211> 24
   <212> DNA
   <213> Künstliche Sequenz
<220>
   <223> Beschreibung der künstlichen Sequenz:
   Oligonukleotid
<400> 13
   ctggagaaga gctacgagct gcct 24
<210> 14
   <211> 24
   <212> DNA
   <213> Künstliche Sequenz
<220>
   <223> Beschreibung der künstlichen Sequenz:
   oligonukleotid
<400> 14
   acaggactcc atgcccagga agga 24